# EUROPEAN PATENT APPLICATION

(11) **EP 4 183 785 A1**
(43) Date of publication of application: **24.05.2023**
(21) Application number: 21899999.3
(22) Date of filing: 30.11.2021
(51) Int. Cl.: C07D 471/04, C07D 487/04, A61K 31/407, A61K 31/437, A61P 35/00

(54) **NOVEL N-HETEROCYCLIC BET BROMODOMAIN INHIBITOR, AND PREPARATION METHOD THEREFOR AND MEDICAL USE THEREOF**

(30) Priority: 01.12.2020 CN 202011386289; 25.12.2020 CN 202011558334; 07.02.2021 CN 202110167605
(71) Applicant: Chengdu Easton Biopharmaceuticals Co., Ltd., Hi-tech District Chengdu, Sichuan 611731 (CN)
(72) Inventor: ZHU, Hui, Chengdu, Sichuan 611731 (CN); ZHANG, Jibing, Chengdu, Sichuan 611731 (CN); ZHANG, Xinlong, Chengdu, Sichuan 611731 (CN); SHU, Tianbo, Chengdu, Sichuan 611731 (CN); ZHANG, Tao, Chengdu, Sichuan 611731 (CN); XIANG, Yao, Chengdu, Sichuan 611731 (CN); WANG, Ying, Chengdu, Sichuan 611731 (CN)
(74) Representative: Manitz Finsterwald Patent- und Rechtsanwaltspartnerschaft mbB
(86) International application number: PCT/CN2021/134454
(87) International publication number: WO 2022/116968

(57) **Abstract**

Provided in the present application are a compound of formula (I), a pharmaceutically acceptable salt thereof, a preparation method thereof, and the medical use thereof. The compound can be used as an agent in the treatment of diseases and conditions, and the diseases and conditions include cancer, inflammatory diseases, autoimmune diseases, etc.

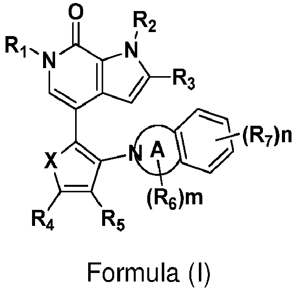

## Description

### TECHNICAL FIELD

The present application is related to the technical field of biomedicine, and in particular, to a novel n-heterocyclic BET bromodomain inhibitor, and a preparation method and a medical use thereof.

### BACKGROUND

Histone acetylation is an important part of epigenetic research. Acetylated histone can activate gene transcription through the action of DNA polymerase, RNA polymerase and transcription factors. Bromodomain and extraterminal domain (BET) family belongs to bromodomain proteins (BRDs), i.e. a class of evolutionarily highly conserved proteins capable of recognizing and binding to the acetylated lysine residues at the tail of histone, and recruiting chromatin-regulating proteins, transcription factors, chromatin remodeling factors, etc., thereby playing an important role in gene transcription regulation and chromatin remodeling, and BRDs are associated with various biological processes such as the growth, the proliferation and differentiation, and the apoptosis and necrosis of cells, and serve as important epigenetic "readers".

So far, it has been found that the human genome encodes a total of 61 bromodomains, which are distributed in 46 different proteins. The BRD family consists of four subtypes, BRD2, BRD3, BRD4, and BRDT, each of which contains two tandem bromodomains (BD1 and BD2) and an extraterminal (ET) domain. The two bromodomains are mainly responsible for recognizing and binding to acetylated lysine residues, and the ET domain interacts with cofactors. The abnormal expression of BET proteins is associated with many diseases. Although the four members of the BET family have similar structures, they are different in their biological functions. Especially, BRD4 is closely associated with various diseases such as cancer and inflammation (Jung M et al. Epigenomics, 2015, 7(3): 487-501.).

Targeting BET proteins is beneficial for the development of new therapeutic strategies targeting cancer, inflammation, and viruses. As present, small molecule inhibitors targeting BET proteins, which are mainly used for the treatment of cancer and autoimmune diseases, have entered the phases of preclinical and clinical research. For example, Picaud et al. (Picaud S et al., Nature, 2010, 468 (7327): 1067-1073.) developed BET small-molecule inhibitor JQ1 in 2010, and corresponding biological experiments show that JQ1 has potential therapeutic value for diseases such as cancer, cardiovascular diseases, human immunodeficiency virus (HIV) infection and inflammation; GSK (WO2011054553A) disclosed that BET small-molecule inhibitor I-BET762 (GSK525762, Nicodeme E et al., Nature, 2010, 468, 7327) is significantly effective in treating inflammation and cancer, and it has entered phase I/II clinical trials now; RVX-208 developed by Resverlogix Corp. (McLure KG et al., PLoS One, 2013, 8, 12: e83190.) is a quinazolinone inhibitor selectively acting on BD2, and clinical data shows that RVX-208 is significantly effective in treating diseases such as coronary syndrome, atherosclerosis and diabetes mellitus, and RVX-208 has entered phase III clinical trials by now; OTX015 developed by OncoEthix (Brand M. et al., ACS Chem Biol, 2015, 10 (1): 22-39.) can act on solid tumors such as nuclear protein in testis (NUT) midline carcinoma (NMC) and prostate cancer in children and young adults, and has entered phase II clinical trials; Incyte disclosed compound INCB-057643 in patent application CN106414442A which has entered phase I clinical trials; and Abbive disclosed a class of selective BET protein inhibitors in patent application WO2017177955A1, and compound ABBV-744 shows better safety in preclinical research, and has entered phase I clinical trials by now.

In summary, BET protein inhibitors have good application prospects as drugs, and there is a continuous clinical demand for the development of new BET protein inhibitors with high efficiency and low toxicity.

### SUMMARY

In the present application, a series of novel BET bromodomain inhibitors with benzazepine heterocycle as the parent nucleus are designed, and by constructing the new parent nucleus, the series of compounds obtained show good biological activities and druggability. In particular, a compound of formula (I) or a pharmaceutically acceptable salt thereof is provided: 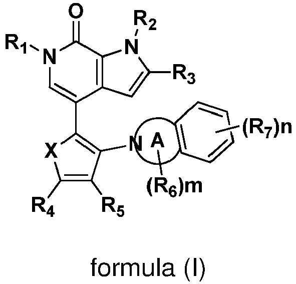 where:
ring A is a 5- to 8-membered saturated or unsaturated N-containing heterocycle, and ring A and its adjacent benzene ring form a fused ring;
X is O or S;
m is 0, 1, 2, 3, 4, 5 or 6; and
n is 0, 1, 2, 3 or 4;
R₁ is hydrogen or a C₁-C₆ alkyl group;
R₂ is hydrogen or a C₁-C₆ alkyl group or a C₃-C₆ cycloalkyl group;
R₃ is hydrogen, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
R₄ is hydrogen, -C(CH₃)₂OH, -CH₂C(CH₃)₂OH, -C(O)Rₓ, -C(O)ORₓ, -C(O)NHRₓ, - C(O)NRₓ₁R_{y1}, -S(O)₂Rₓ, -S(O)₂NRₓ₁R_{y1} or -N(Rₓ)S(O)₂R_{y};
R₅ is hydrogen, halogen, a cyano group, a hydroxyl group, an amino group, a nitro group, a C₁-C₆ alkyl group, a C₃-C₆ cycloalkyl group, a C₁-C₃ alkoxy group or a C₁-C₃ haloalkyl group;
R₆ is hydrogen, halogen, a cyano group, a hydroxyl group, an amino group, a C₁-C₆ alkyl group, a C₃-C₆ cycloalkyl group, a C₁-C₃ alkoxy group or a C₁-C₃ haloalkyl group, or two R₆ groups on the same carbon atom can form a C₃-C₈ spiro group together;
R₇ is hydrogen, halogen, a cyano group, a hydroxyl group, an amino group, a nitro group, a C₁-C₆ alkyl group, a C₃-C₆ cycloalkyl group, a C₁-C₃ alkoxy group, a C₁-C₃ haloalkyl group, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
Rₓ and R_{y} are each independently selected from hydrogen, a C₁-C₆ alkyl group or a C₃-C₆ cycloalkyl group; and
Rₓ₁ and R_{y1} are each independently selected from hydrogen, a C₁-C₆ alkyl group or a C₁-C₆ heteroalkyl group, or Rₓ₁, R_{y1} and a nitrogen atom are connected to form a 3- to 8-membered ring.

In some preferable embodiments, ring A is a 5- to 6-membered saturated or unsaturated N-containing heterocycle; and for example, ring A can be pyrrole, dihydropyrrole, tetrahydropyrrole, pyridine, tetrahydropyridine, piperidine, or the like.

In some preferable embodiments, the fused ring formed by ring A and its adjacent benzene ring is selected from: 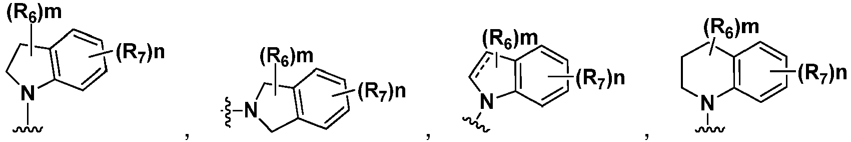 or

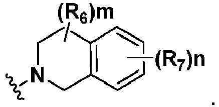

In some preferable embodiments, when m is 2, 3, 4, 5 or 6, multiple R₆ groups can be the same or different; and when n is 2, 3 or 4, multiple R₇ groups can be the same or different.

In some preferable embodiments, R₁ is hydrogen or a C₁-C₄ alkyl group, and preferably, hydrogen or a C₁-C₃ alkyl group.

In some preferable embodiments, R₁ is hydrogen, a methyl group, an ethyl group, a n-propyl group, or an isopropyl group.

In some preferable embodiments, R₂ is hydrogen or a C₁-C₃ alkyl group.

In some preferable embodiments, R₂ is hydrogen, a methyl group, an ethyl group, a n-propyl group, an isopropyl group or a cyclopropyl group.

In some preferable embodiments, R₃ is hydrogen, -C(O)Rₓ, -C(O)ORₓ, - C(O)NHRₓ, -C(O)NRₓ₁R_{y1}, -S(O)₂NRₓ₁R_{y1}, -N(Rₓ)S(O)₂R_{y} or -NRₓ₁R_{y1};
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring:

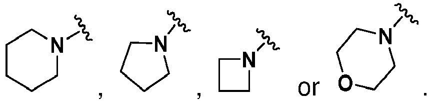

In some preferable embodiments, R₃ is hydrogen, -C(O)Rₓ, -C(O)ORₓ, - C(O)NHRₓ, -C(O)NRₓ₁R_{y1}, -S(O)₂NRₓ₁R_{y1}, -N(Rₓ)S(O)₂R_{y} or -NRₓ₁R_{y1};
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group.

In some preferable embodiments, R₃ is hydrogen, -C(O)ORₓ, -C(O)NHRₓ or -C(O)NRₓ₁R_{y1}, Rₓ is selected from hydrogen or a C₁-C₆ alkyl group (for example, a C₁-C₄ alkyl group), and Rₓ₁ and R_{y1} are each independently selected from hydrogen or a C₁-C₆ alkyl group (for example, a C₁-C₄ alkyl group).

In some preferable embodiments, R₄ is hydrogen, -C(CH₃)₂OH, - CH₂C(CH₃)₂OH, -C(O)Rₓ, -C(O)ORₓ, -C(O)NHRₓ, -C(O)NRₓ₁R_{y1}, -S(O)₂Rₓ, - S(O)₂NRₓ₁R_{y1} or -N(Rₓ)S(O)₂R_{y}:
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring:

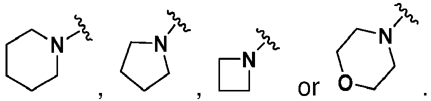

In some preferable embodiments, R₄ is hydrogen, -C(CH₃)₂OH, - CH₂C(CH₃)₂OH, -C(O)Rₓ, -C(O)ORₓ, -C(O)NHRₓ, -C(O)NRₓ₁R_{y1}, -S(O)₂Rₓ, - S(O)₂NRₓ₁R_{y1} or -N(Rₓ)S(O)₂R_{y}.
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group.

In some preferable embodiments, R₄ is hydrogen, -C(CH₃)₂OH, - CH₂C(CH₃)₂OH, -C(O)ORₓ or -C(O)NHRₓ, and Rₓ is hydrogen or a methyl group.

In some preferable embodiments, R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group.

In some preferable embodiments, R₅ is hydrogen, a hydroxyl group, an amino group, a methyl group or a methoxy group.

In some preferable embodiments, R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃-C₆ spiro group.

In some preferable embodiments, R₆ is hydrogen, halogen, a hydroxyl group, an amino group, a C₁-C₄ alkyl group, a C₃-C₄ cycloalkyl group or a C₁-C₃ alkoxy group, or two R₆ groups on the same carbon atom form a C₃-C₄ spiro group.

In some preferable embodiments, R₆ is hydrogen, halogen, a hydroxyl group, an amino group, a methyl group, a cyclopropyl group or a methoxy group, or two R₆ groups on the same carbon atom form a C₃ spiro group.

In some preferable embodiments, R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group, a trifluoromethyl group, -C(O)Rₓ, -C(O)ORₓ, -C(O)NHRₓ, -C(O)NRₓ₁R_{y1}, - S(O)₂NRₓ₁R_{y1}, -N(Rₓ)S(O)₂R_{y} or -NRₓ₁R_{y1};
Rₓ and R_{y} are each independently selected from hydrogen, a methyl group, an ethyl group, an isopropyl group or a cyclopropyl group; and
Rₓ₁ and R_{y1} are independently hydrogen, a methyl group, an ethyl group, an isopropyl group or a cyclopropyl group, or Rₓ₁, R_{y1} and a nitrogen atom are connected to form the following ring:

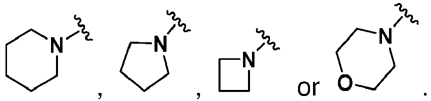

In some preferable embodiments, R₇ is hydrogen, halogen, a cyano group, a hydroxyl group, an amino group, a C₁-C₄ alkyl group, a C₁-C₃ alkoxy group, a C₁-C₃ haloalkyl group, -C(O)Rₓ, -C(O)ORₓ, -C(O)NHRₓ or -C(O)NRₓ₁R_{y1}, Rₓ is hydrogen, a methyl group or an ethyl group, and Rₓ₁ and R_{y1} are independently hydrogen or a methyl group.

In some preferable embodiments, R₇ is hydrogen, halogen, a cyano group, a hydroxyl group, an amino group, a C₁-C₄ alkyl group, a C₁-C₃ alkoxy group or a C₁-C₃ haloalkyl group.

In some preferable embodiments, R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group or a trifluoromethyl group.

In some preferable embodiments, X is S.

In some preferable embodiments, R₃ is hydrogen, -C(O)Rₓ, -C(O)ORₓ, - C(O)NHRₓ, -C(O)NRₓ₁R_{y1}, -S(O)₂NRₓ₁R_{y1}, -N(Rₓ)S(O)₂R_{y} or -NRₓ₁R_{y1};
R₄ is hydrogen, -C(CH₃)₂OH, -CH₂C(CH₃)₂OH, -C(O)Rₓ, -C(O)ORₓ, -C(O)NHRₓ, - C(O)NRₓ₁R_{y1}, -S(O)₂Rₓ, -S(O)₂NRₓ₁R_{y1} or -N(Rₓ)S(O)₂R_{y};
R_{y} is hydrogen, halogen, a cyano group, a hydroxyl group, an amino group, a nitro group, a C₁-C₆ alkyl group, a C₃-C₆ cycloalkyl group, a C₁-C₃ alkoxy group, a C₁-C₃ haloalkyl group, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring:

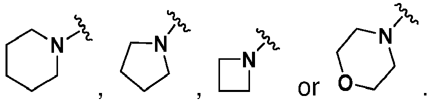

In some preferable embodiments, R₁ is hydrogen, a methyl group, an ethyl group, a n-propyl group or an isopropyl group;
R₂ is hydrogen, a methyl group, an ethyl group, a n-propyl group, an isopropyl group or a cyclopropyl group;
R₃ is hydrogen, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
R₄ is hydrogen, -C(CH₃)₂OH, -CH₂C(CH₃)₂OH, -C(O)Rₓ, -C(O)ORₓ, -C(O)NHRₓ, - C(O)NRₓ₁R_{y1}, -S(O)₂Rₓ, -S(O)₂NRₓ₁R_{y1} or -N(Rₓ)S(O)₂R_{y};
R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group;
R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃-C₆ spiro group;
R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group, a trifluoromethyl group, -C(O)Rₓ, -C(O)ORₓ, -C(O)NHRₓ, -C(O)NRₓ₁R_{y1}, -S(O)₂NRₓ₁R_{y1}, -N(Rₓ)S(O)₂R_{y} or - NRₓ₁R_{y};
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring: 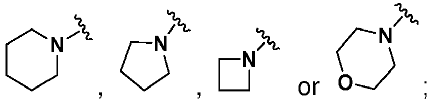
m is 0, 1, 2, 3, 4, 5 or 6; and
n is 0, 1, 2, 3 or 4;
when m is 2, 3, 4, 5 or 6, multiple R₆ groups can be the same or different; and
when n is 2, 3 or 4, multiple R₇ groups can be the same or different.

In some preferable embodiments, X is O or S;
R₁ is hydrogen, a methyl group, an ethyl group, a n-propyl group or an isopropyl group;
R₂ is hydrogen, a methyl group, an ethyl group, a n-propyl group, an isopropyl group or a cyclopropyl group;
R₃ is -C(O)NHRₓ;
R₄ is -CH₂C(CH₃)₂OH, -C(CH₃)₂OH or -C(O)ORₓ;
Rₓ is selected from hydrogen, a methyl group, an ethyl group, an isopropyl group or a cyclopropyl group;
R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group;
R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃ spiro group;
R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group or a trifluoromethyl group;
m is 0, 1 or 2; and
n is 0, 1 or 2.

In some preferable embodiments, X is S;
R₁ is a methyl group;
R₂ is hydrogen or a cyclopropyl group;
R₃ is -C(O)NHCH₃ or -C(O)NHCH₂CH₃;
R₄ is -CH₂C(CH₃)₂OH, -C(O)OCH₂CH₃ or -C(CH₃)₂OH;
R₅ is hydrogen, fluorine, chlorine, bromine, iodine or a methyl group;
R₆ is hydrogen, fluorine, chlorine, bromine, iodine, a methyl group, an ethyl group, an isopropyl group or a cyclopropyl group, or two R₆ groups on the same carbon atom form a C₃ spiro group;
R₇ is hydrogen, fluorine, chlorine, bromine, iodine, a cyano group, a methyl group, an ethyl group, an isopropyl group, a cyclopropyl group, a methoxy group, an ethoxy group or a trifluoromethyl group;
m is 0, 1 or 2; and
n is 0, 1 or 2.

In some preferable embodiments, ring A is a 5- to 6-membered saturated or unsaturated N-containing heterocycle, preferably, pyrrole, dihydropyrrole, tetrahydropyrrole, pyridine, tetrahydropyridine or piperidine, and more preferably, a fused ring formed by ring A and its adjacent benzene ring is selected from: 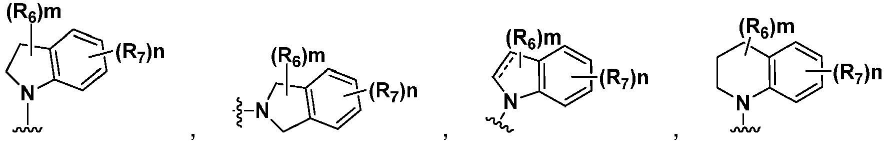 or 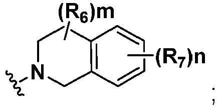
X is O or S;
R₁ is hydrogen or a C₁-C₄ alkyl group, and preferably, hydrogen or a C₁-C₃ group;
R₂ is hydrogen or a C₁-C₃ alkyl group;
R₃ is hydrogen, -C(O)ORₓ, -C(O)NHRₓ or -C(O)NRₓ₁R_{y1};
R₄ is hydrogen, -C(CH₃)₂OH, -CH₂C(CH₃)₂OH, -C(O)ORₓ or -C(O)NHRₓ;
R₅ is hydrogen, a hydroxyl group, an amino group, a methyl group or a methoxy group;
R₆ is hydrogen, halogen, a hydroxyl group, an amino group, a C₁-C₄ alkyl group, a C₃-C₄ cycloalkyl group or a C₁-C₃ alkoxy group, or two R₆ groups on the same carbon atom form a C₃-C₄ spiro group; and preferably, R₆ is hydrogen, halogen, a hydroxyl group, an amino group, a methyl group, a cyclopropyl group or a methoxy group, or two R₆ groups on the same carbon atom form a C₃ spiro group;
R₇ is hydrogen, halogen, a cyano group, a hydroxyl group, an amino group, a C₁-C₄ alkyl group, a C₁-C₃ alkoxy group, a C₁-C₃ haloalkyl group, -C(O)Rₓ, -C(O)ORₓ, - C(O)NHRₓ or -C(O)NRₓ₁R_{y1}; and preferably, R₇ is hydrogen, halogen, a cyano group, a hydroxyl group, an amino group, a C₁-C₄ alkyl group, a C₁-C₃ alkoxy group or a C₁-C₃ haloalkyl group; or
Rₓ is selected from hydrogen or a C₁-C₆ alkyl group, and preferably, hydrogen or a C₁-C₄ group;
Rₓ₁ and R_{y1} are each independently selected from hydrogen or a C₁-C₆ alkyl group, and preferably, hydrogen or a C₁-C₄ group;
m is 0, 1, 2 or 3; and
n is 0, 1, 2 or 3.

In some preferable embodiments, ring A is a 5- to 6-membered saturated or unsaturated N-containing heterocycle;
X is S;
R₁ is a methyl group;
R₂ is hydrogen or a cyclopropyl group;
R₃ is -C(O)NHCH₃ or -C(O)NHCH₂CH₃;
R₄ is -CH₂C(CH₃)₂OH, -C(O)OCH₂CH₃ or -C(CH₃)₂OH;
R₅ is hydrogen, fluorine, chlorine, bromine, iodine or a methyl group;
R₆ is hydrogen, fluorine, chlorine, bromine, iodine, a methyl group, an ethyl group, an isopropyl group or a cyclopropyl group, or two R₆ groups on the same carbon atom form a C₃ spiro group;
R₇ is hydrogen, fluorine, chlorine, bromine, iodine, a cyano group, a methyl group, an ethyl group, an isopropyl group, a cyclopropyl group, a methoxy group, an ethoxy group or a trifluoromethyl group;
m is 0, 1 or 2; and
n is 0, 1 or 2.

In some preferable embodiments, ring A is a 5- to 6-membered saturated or unsaturated N-containing heterocycle;
X is S;
R₁ is a methyl group;
R₂ is hydrogen;
R₃ is -C(O)NHCH₂CH₃;
R₄ is -C(CH₃)₂OH;
R₅ is hydrogen;
R₆ is hydrogen;
R₇ is hydrogen, fluorine, a cyano group, a methyl group or a trifluoromethyl group;
m is 0 or 1; and
n is 0 or 1.

In some preferable embodiments, ring A is a 5-membered saturated or unsaturated N-containing heterocycle;
X is S;
R₁ is a methyl group;
R₂ is hydrogen;
R₃ is -C(O)NHCH₂CH₃;
R₄ is -C(CH₃)₂OH;
R₅ is hydrogen;
R₆ is hydrogen;
R₇ is hydrogen or fluorine;
m is 0 or 1; and
n is 0 or 1.

The present application further involves any combination of the above embodiments and preferable embodiments.

In some preferable embodiments, the compound is selected from:

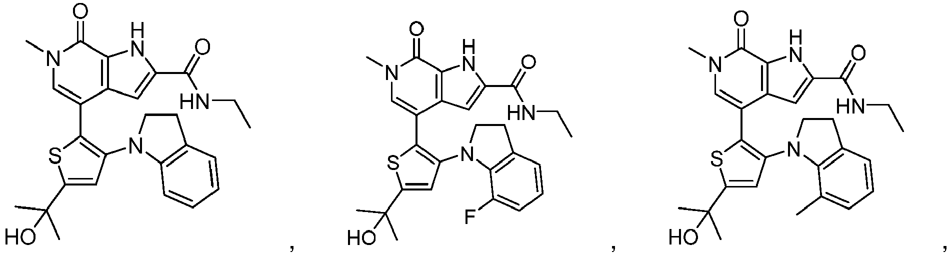

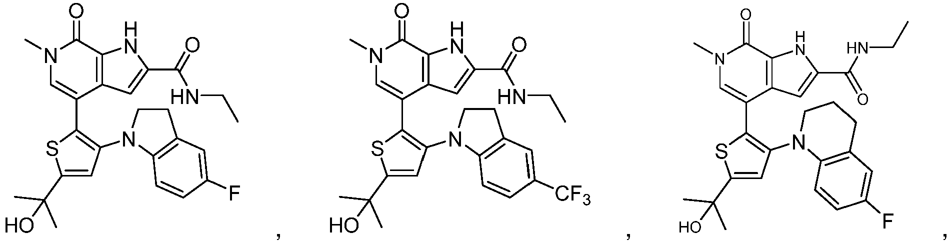

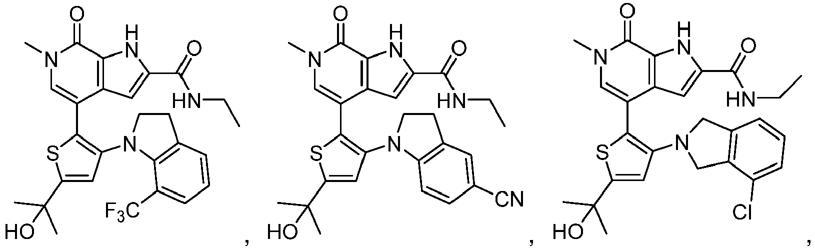

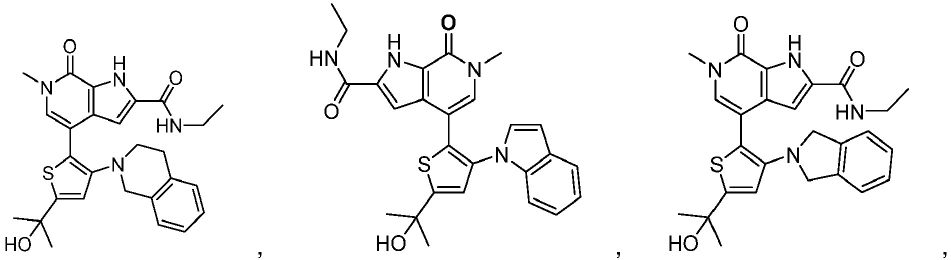

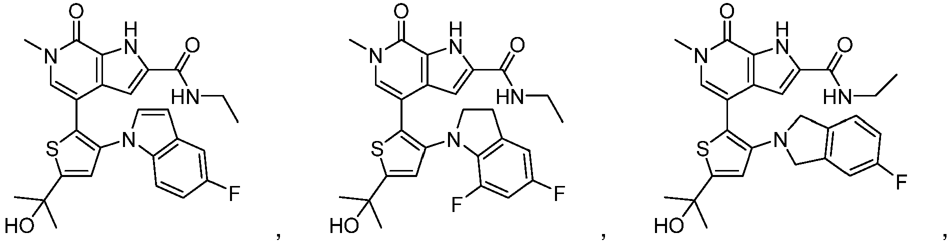

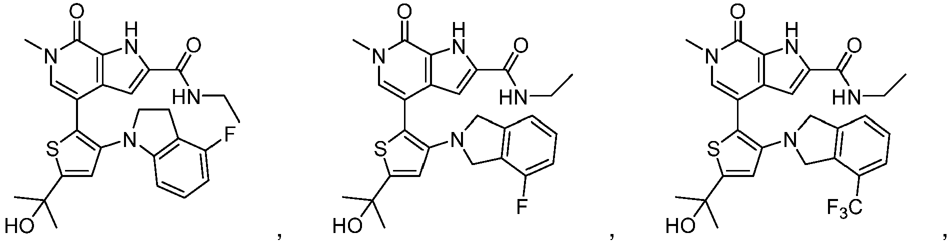

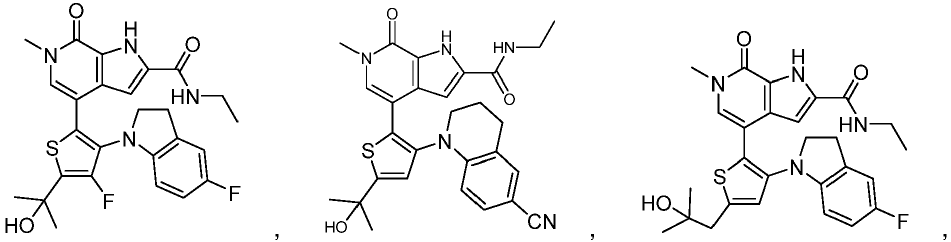

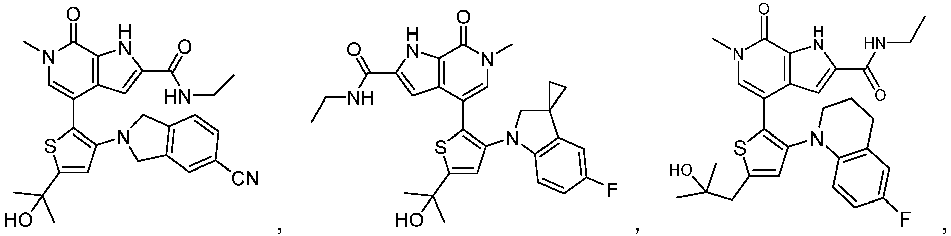

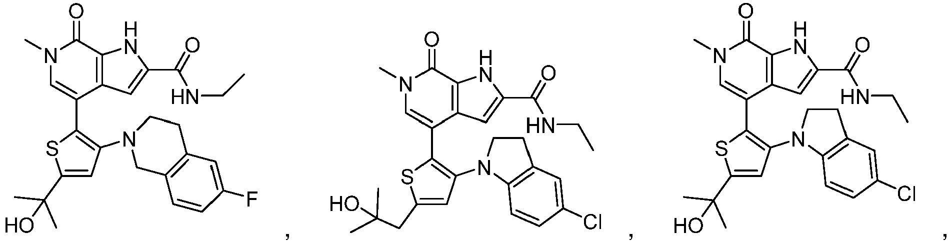

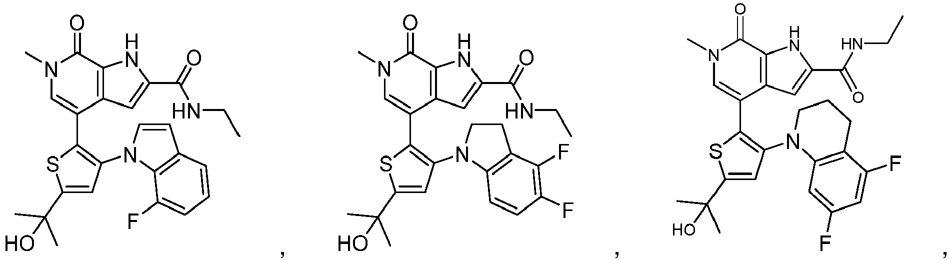

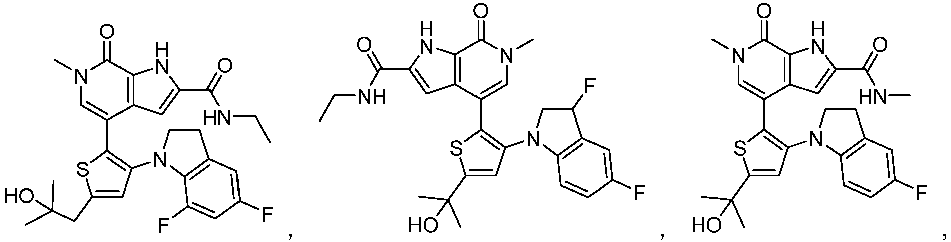

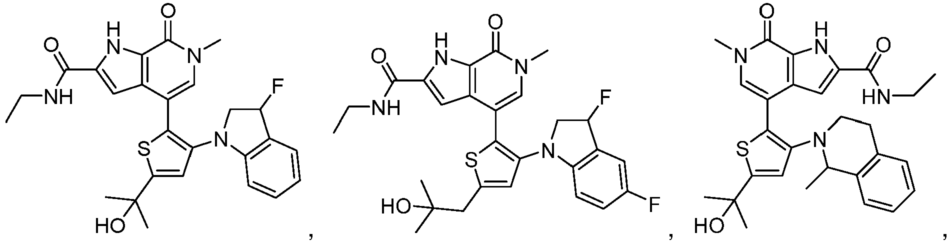

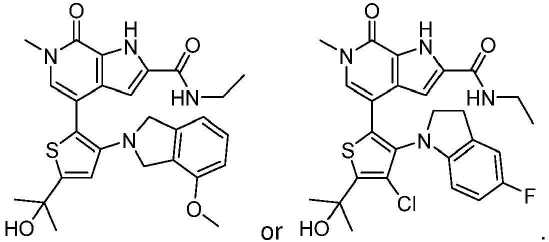

According to another aspect, the present application further provides a method for synthesizing the compound of formula (I), including but not limited to steps of: 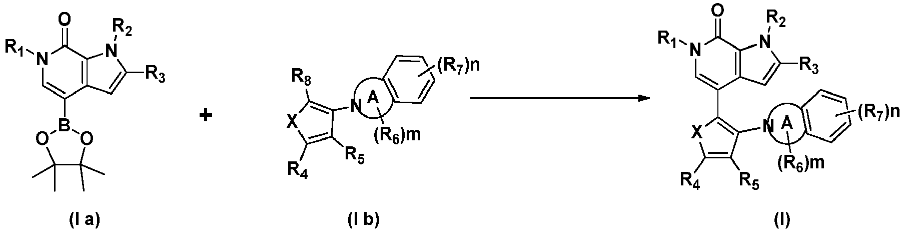 , or 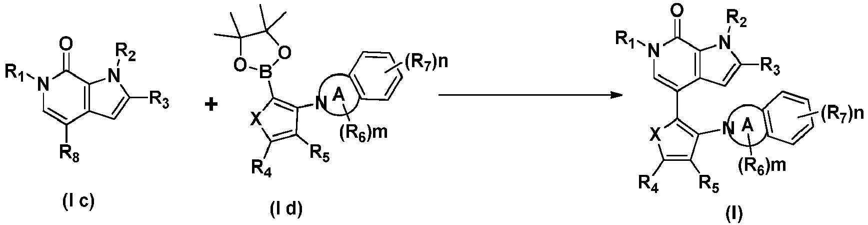 subjecting a compound of formula (Ia) and a compound of formula (Ib), or a compound of formula (Ic) and a compound of formula (Id) to a catalytic coupling reaction to obtain the compound of formula (I), where each of the groups is defined as above, and R₈ is selected from C!, Br or I.

In some preferable embodiments, the catalytic coupling reaction is carried out in 1 ,4-dioxane in the presence of a ligand (for example, Xphos or Xantphos), an inorganic base (for example, cesium carbonate) and a palladium catalyst (for example, Pd₂(dba)₃ catalyst).

In some preferable embodiments, the catalytic coupling reaction is carried out in an inert atmosphere (for example, a nitrogen atmosphere or an argon atmosphere) at a temperature of 80°C to 120°C (for example, 105°C) for 6 to 10 hours (for example, 8 hours).

According to another aspect, the present application further provides intermediate (lb): 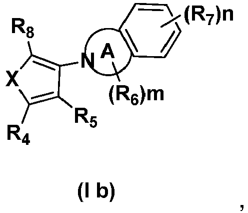 where each of the groups is defined as above, and R₈ is selected from C!, Br or I.

According to another aspect, the present application further provides intermediate (Id): 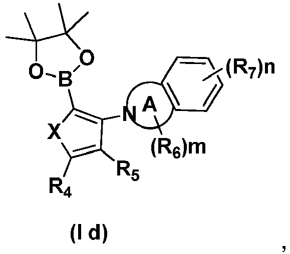 where each of the groups is defined as above.

According to another aspect, the present application further involves a pharmaceutical composition comprising the compound of formula (I) or a pharmaceutically acceptable salt thereof. In some embodiments, the pharmaceutical composition in the present application further includes at least one pharmaceutically acceptable excipient.

According to another aspect, the present application further involves use of the above compound of formula (I) or a pharmaceutically acceptable salt thereof, or the above pharmaceutical composition in the manufacture of a medicament for prevention and/or treatment of a BET protein-associated disease.

According to another aspect, the present application further involves use of the above compound of formula (I) or a pharmaceutically acceptable salt thereof, or the above pharmaceutical composition in prevention and/or treatment of a BET protein-associated disease.

According to another aspect, the present application further involves a method for preventing and/or treating a BET protein-associated disease, comprising: administering the above compound of formula (I) or a pharmaceutically acceptable salt thereof, or the above pharmaceutical composition to a subject in need.

According to another aspect, the present application further involves the above compound of formula (I) or a pharmaceutically acceptable salt thereof, or the above pharmaceutical composition for use in prevention and/or treatment of a BET protein-associated disease.

According to another aspect, the present application further involves use of the above compound of formula (I) or a pharmaceutically acceptable salt thereof, or the above pharmaceutical composition in the manufacture of a BET protein inhibitor.

According to another aspect, the present application further involves the above compound of formula (I) or a pharmaceutically acceptable salt thereof, or the above pharmaceutical composition for use as a BET protein inhibitor.

In some embodiments, the BET protein-associated disease includes, but is not limited to, a tumor disease, an inflammatory disease, an autoimmune disease or a viral infection.

In some embodiments, the tumor disease includes, but is not limited to, a non-solid tumor (for example, leukemia) or a solid tumor (carcinoma and sarcoma), such as acute leukemia, acute lymphoblastic leukemia, acute myeloid leukemia (comprising monocytic leukemia, myeloblastic leukemia, myelomonocytic leukemia or promyelocytic leukemia), acute T-cell leukemia, B-cell acute lymphoblastic leukemia, angiosarcoma, astrocytoma, basal cell carcinoma, cholangiocarcinoma, bladder cancer, brain cancer, breast cancer, bronchial cancer, prostate cancer, cervical cancer, chondrosarcoma, chordoma, choriocarcinoma, chronic leukemia, chronic lymphocytic leukemia, chronic myeloid leukemia and chronic myelocytic leukemia, chronic myelogenous leukemia, colon cancer, colorectal cancer or non-small cell lung cancer.

The present application discloses a BET protein inhibitor with a novel structure represented by formula (I), which has better BET protein inhibition activity and good pharmacokinetic properties, and also shows good in vivo tumor inhibition activity with a lower risk of hERG cardiotoxicity, and thus, the BET protein inhibitor is a new generation of BET protein inhibitor with high efficacy and low toxicity.

### DETAILED DESCRIPTION

The technical solutions in the embodiments of the present application will be clearly and completely described below. Apparently, the embodiments described are only a part rather than all of the embodiments of the present application. All other embodiments obtained by a person of ordinary skill in the art based on the embodiments of the present application without making inventive efforts shall fall within the protection scope of the present application.

### Definitions and Explanation

Unless otherwise stated, the terms used in the present application are defined as below. In the case that a specific term is not specifically defined, it should not be considered as indeterminate or unclear, but should be understood based on its common meaning in the art. A commodity name used herein means to refer to a corresponding commodity or an active ingredient thereof.

In the present application, in the case that a covalent bond in a structural unit or a group is not connected to a specific atom, it means the covalent bond can be connected to any atom in the structural unit or group, provided that a valence bond connection rule is not violated.

Unless otherwise stated, the term "N-containing heterocycle" used herein includes a non-aromatic carbocycle or cycloalkane containing one or more (for example, 1 to 3 or 1 or 2) nitrogen atoms, and the N-containing heterocycle is optionally substituted by one or more (for example, 1 to 3) suitable substituents (for example, halogen, a cyano group, a hydroxyl group, an amino group or a C₁-C₆ alkyl group). For example, the "N-containing heterocycle" includes a 5- to 10-membered non-aromatic carbocycle or cycloalkane containing 1 nitrogen atom, which is optionally partially or fully saturated.

Unless otherwise stated, the expression "ring A together with its adjacent benzene ring forms a fused ring" used herein means that ring A as a N-containing heterocycle forms a ring structure with its adjacent benzene ring, and the N-containing heterocycle and the benzene ring are each independently optionally substituted by one or more (for example, 1 to 3) suitable substituents (for example, halogen, a cyano group, a hydroxyl group, an amino group or a C₁-C₆ alkyl group); and exemplarily, the fused ring includes: 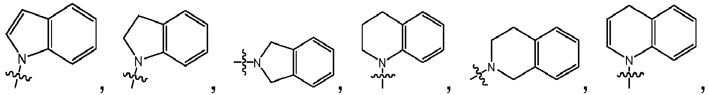 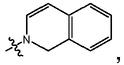 or the like.

Unless otherwise stated, the term "Cₘ to Cₙ" used herein means that m to n carbon atoms (n is greater than m, and both n and m are integers) are included. For example, "C₁ to C₆" means that 1 to 6 carbon atoms, such as 1, 2, 3, 4, 5 or 6 carbon atoms are included.

Unless otherwise stated, the term "alkyl group" used herein refers to a saturated hydrocarbyl group consisting of only carbon atoms and hydrogen atoms, including but not limited to, a C₁ to C₆ alkyl group, a C₁ to C₅ alkyl group, a C₁ to C₄ alkyl group, a C₁ to C₃ alkyl group, a C₁ or C₂ alkyl group and a C₁ alkyl group. As a non-limited example, the alkyl group may be a linear or branched saturated hydrocarbyl group selected from a methyl group, an ethyl group, an n-propyl group, an isopropyl group, an n-butyl group, an isobutyl group, a sec-butyl group, a tert-butyl group, an n-pentyl group and seven other isomers thereof, and an n-hexyl group and sixteen other isomers thereof. For example, a C₁ to C₇ alkyl group includes a methyl group, an ethyl group, a propyl group, a butyl group, a pentyl group, a hexyl group, a heptyl group and all the isomers thereof.

Unless otherwise stated, the term "cycloalkyl group" used herein refers to a completely saturated carbocycle in the form of a monocyclic, bridged, or spiro ring. Unless otherwise stated, the cycloalkyl group herein may be a 3- to 10-membered ring, such as a 3-membered ring, a 4-membered ring, a 5-membered ring or a 6-membered ring. Non-limiting examples of the cycloalkyl group include a cyclopropyl group, a cyclobutyl group, a cyclopentyl group, a cyclohexyl group, and the like.

Unless otherwise stated, the term "alkoxy group" used herein refers to -O-alkyl.

Unless otherwise stated, the term "halogen" used herein refers to fluorine, chlorine, bromine, or iodine.

Unless otherwise stated, the term "haloalkyl group" used herein refers to an alkyl group as defined above which is substituted by one or more (preferably, 1 to 5, for example, 1, 2, 3, 4 or 5) halogen atoms. The haloalkyl group includes a monohaloalkyl group, a dihaloalkyl group, a trihaloalkyl group or a perhaloalkyl group, such as a chloromethyl group, a dichloromethyl group, a difluoromethyl group, a dibromomethyl group, a trifluoromethyl group, a 2,2,2-trifluoroethyl group, a perfluoroethyl group or a 2,2,2-trifluoro-1 ,1-dichloroethyl group.

Unless otherwise stated, the term "heteroalkyl group" used herein refers to an alkyl group as defined above in which an alkane chain is connected to a heteroatom selected from N, O or S. For example, a C₁ heteroalkyl group means an alkyl group in which an alkane chain is connected to a heteroatom selected from N, O or S and which contains 1 carbon atom.

As used herein, the term "substitution" means that one or more (for example, one, two, three or four) hydrogen atoms on a specified atom are substituted by selection(s) of specified groups, provided that a normal valence of the specified atom is not exceeded and such substitution results in a stable compound. A combination of substituents and/or variables are allowable only if such combination results in a stable compound.

If a substituent is described as being "optionally substituted", the substituent may be: (1) unsubstituted or (2) substituted. If a carbon atom of a substituent is described as being optionally substituted by one or more of a list of substituents, one or more hydrogen atoms on the carbon atom may be optionally individually and/or jointly substituted by independently selected substituent(s).

Herein, the term "subject" is equivalent to "patient" and "individual", and refers to a human or a non-human animal (a mammal such as a primate or a rodent). "Mammal" includes human, livestock (such as laboratory mammals and household pets, such as cats, dogs, pigs, rams, cattle, sheep, goats, horses and rabbits), and non-domesticated mammals, such as wild mammals.

The term "treatment" means administration of the compound or formulation of the present application to mitigate or eliminate a disease or one or more symptoms associated with the disease, including suppressing the progression of and alleviating the disease or disorder.

The term "prevention" means administration of the compound or formulation of the present application to prevent a disease or one or more symptoms associated with the disease, including prevention of the occurrence of a disease or disorder in a subject, especially when the subject is predisposed to but have not yet been diagnosed with the disease or disorder.

The term "therapeutically effective amount" refers to an amount of the compound of the present application that is sufficient to: (i) treat or prevent a disease, condition or disorder; (ii) alleviate, improve or eliminate one or more symptoms of a disease, condition or disorder; or (iii) delay onset of one or more symptoms of a disease, condition or disorder. The "therapeutically effective amount" depends on the compound, the disease and its severity, the way of administration, and the condition of the subject to be treated, and can be routinely determined by persons skilled in the art based on their own knowledge and this disclosure.

The term "pharmaceutically acceptable" means that the compounds, materials, compositions and/or formulations are suitable for use in contact with human and animal tissues without excessive toxicity, stimulation, allergic reactions or other problems or complications based on reliable medical judgment, and commensurate with a reasonable benefit-risk ratio.

A pharmaceutically acceptable salt may, for example, refer to an acid addition salt or a base addition salt formed by the compound of formula (I) and a pharmaceutically acceptable free acid or free base. The pharmaceutically acceptable salt is, for example, hydrochloride, nitrate, phosphate, sulfate, hydrobromide, hydroiodide, nitrite, phosphite, acetate, benzoate, citrate, lactate, maleate, gluconate, methanesulfonate, benzenesulfonate, p-toluenesulfonate, tartrate, fumarate, malate, oxalate, succinate, a sodium salt, a potassium salt, a calcium salt, an ammonium salt or a magnesium salt.

The term "pharmaceutical composition" refers to a mixture formed by the compound of the present application or a salt thereof and a pharmaceutically acceptable excipient. The pharmaceutical composition is provided for the purpose of facilitating administration of the compound of the present application to an organism.

The term "pharmaceutically acceptable excipient" refers to a pharmaceutical excipient that causes no obvious stimulation to the organism and does not impair the biological activities and performances of the active compound. The excipient herein can be any pharmaceutically acceptable excipient, including, but not limited to, for example, a solvent, a propellant, a solubilizer, a co-solvent, an emulsifier, a colorant, a disintegrant, a filler, a lubricant, a wetting agent, an osmotic pressure regulator, a stabilizer, a glidant, a flavoring agent, a preservative, a suspending agent, an antioxidant, a penetration enhancer, a pH regulator, a surfactant or a diluent. For other available pharmaceutically acceptable pharmaceutical excipients, see, for example, "Handbook of Pharmaceutical Excipients" (4th Edition), R.C. Luo et al., translated by Zheng Zemin, 2005, Chemical Industry Press.

The words "comprise", "include", "contain" and their equivalents should be understood in an open and non-exclusive way, that is, "including but not limited to", which means that other unspecified elements, components and steps may be encompassed in addition to the listed elements, components and steps.

The compound of the present application may have stereoisomers. Unless otherwise stated, the stereoisomers mentioned herein include a geometric isomer and an enantiomer. All these isomers and their mixtures fall within the scope of the present application.

The pharmaceutical composition and the compound of the present application can be prepared into any suitable formulation, for example, a solid, semisolid, liquid or gaseous formulation, such as a tablet, a pill, a capsule, powder, a granule, an ointment, an emulsion, a suspending agent, a suppository, an injection, an inhalant, a gel, a microsphere and an aerosol. The formulation can be produced by a well-known method in the art, for example, a conventional mixing method, a dissolving method, a pellet method, a granulation method, a dragee-making method, a pulverizing method, an emulsifying method or a freeze-drying method.

Typical ways of administration of the compound of the present application or a pharmaceutically acceptable salt thereof or the pharmaceutical composition include, but are not limited to, oral, rectal, topical, inhalation, parenteral, sublingual, intravaginal, intranasal, intraocular, intraperitoneal, intramuscular, subcutaneous and intravenous administrations.

Herein, unless otherwise explicitly stated in the context, a singular term encompasses a plural referent, and vice versa. Similarly, unless otherwise explicitly stated in the context, the word "or" is intended to include "and".

Unless otherwise stated, herein, a parameter value describing a content, physicochemical property, a reaction condition or the like of a component should be understood as being defined with the term "about" in all cases. When the term "about" is used for description in the present application, it means the existence of a tolerance which varies in a range of, for example, ±5%, such as ±1% or ±0.1% of a certain value.

All patents, patent applications, and other identified publications are hereby expressly incorporated herein by reference for the purpose of description and disclosure. These publications are provided only because they are disclosed prior to the filing date of the present application. All statements about dates of these documents or descriptions of the contents of these documents are based on information available to the applicant and cannot be construed as any admission of correctness of the dates of these documents or the contents of these documents. Further, any citation of these publications herein cannot be construed as an admission that the publications become a part of the common general knowledge in the art in any country.

### General Reaction Scheme 1

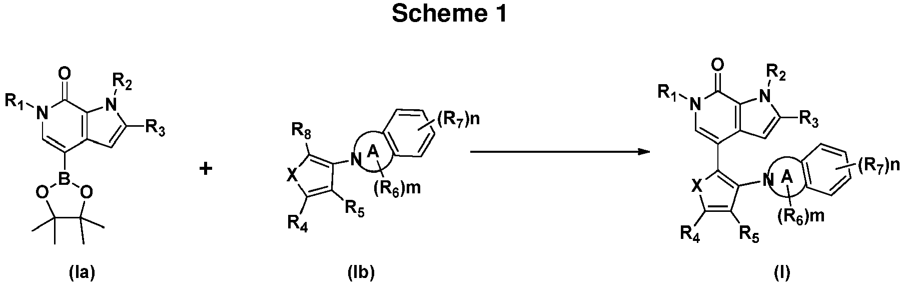

The compound of formula (I) can be prepared according to the general reaction scheme 1 (Scheme 1), where R₁ to R₇, m, n and ring A are defined as above, and R₈ is selected from CI, Br or I. As shown in the general reaction scheme 1, boronate of intermediate (Ia) (synthesized in the method described in WO2017177955A1) and intermediate (lb) react under Suzuki coupling conditions (N. Miyama and A. Suzuki, Chem. Rev. 1995, 95: 2457-2483, J. Organomet. Chem. 1999, 576: 147-148) to obtain the compound of formula (I).

### General Reaction Scheme 2

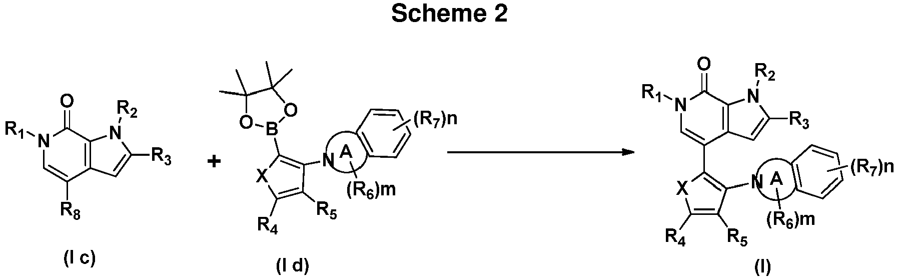

Alternatively, the compound of formula (I) can be prepared according to the general reaction scheme 2 (Scheme 2), where R₁ to R₇, m, n and ring A are defined as above, and R₈ is selected from C!, Br or I. As shown in the general reaction scheme 2, intermediate (Ic) (synthesized in the method described in WO2017177955A1) and boronate of intermediate (Id) react under Suzuki coupling conditions (N. Miyama and A. Suzuki, Chem. Rev. 1995, 95: 2457-2483, J. Organomet. Chem. 1999, 576: 147-148) to obtain the compound of formula (I).

The structure of the compound is determined by mass spectrometry (MS) or ¹H nuclear magnetic resonance (¹H NMR) spectroscopy.

Shift (δ) in ¹H nuclear magnetic resonance (¹H NMR) spectroscopy is expressed in parts per million (ppm); ¹H nuclear magnetic resonance (¹HNMR) spectroscopy is measured with Bruker AVANCE-400 nuclear magnetic resonance spectrometer, with deuterated dimethyl sulfoxide (DMSO-d₆) as a solvent, tetramethylsilane (TMS) as an internal standard substance, and the chemical shift is expressed in a unit of 10⁻⁶ (ppm).

The mass spectrometry (MS) is measured with FINNIGAN LCQAd (ESI) mass spectrometer (manufacturer: Therm, model: Finnigan LCQ advantage MAX).

Thin layer chromatography (TLC) is performed using Yantai Huanghai HSGF254 or Qingdao GF254 silica gel plate.

Column chromatography is generally performed using 200- to 300-mesh Yantai Huanghai silica gel as a carrier.

The term "nitrogen protection" in the present application means that, for example, a reaction flask is connected to a nitrogen balloon with a capacity of 1L, so that the reaction is carried out in a nitrogen atmosphere.

Unless otherwise specified in the present application, the solution used in the reaction in the examples of the present application is aqueous solution.

The term "room temperature" in the present application means that the temperature is between 10°C and 25°C.

### Synthesis of intermediate (la-1):

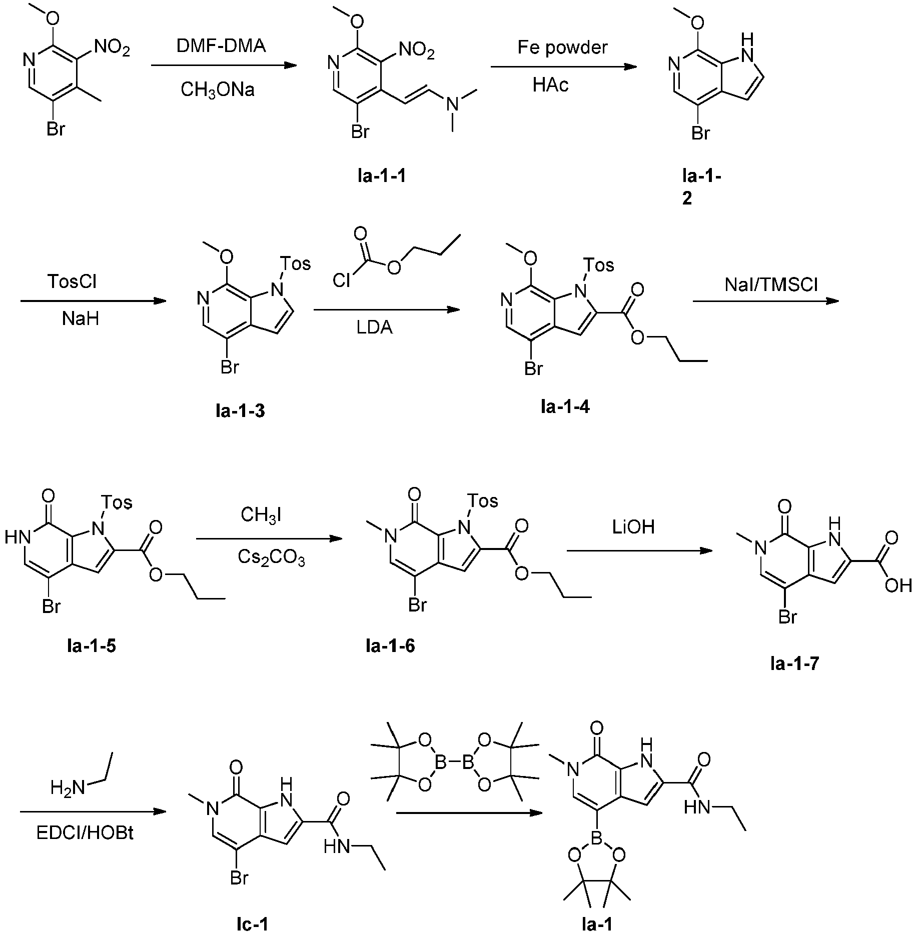

### Step 1: 2-(5-bromo-2-methoxy-3-nitropyridin-4-yl)-N,N-dimethylmethylene-2-amine (la-1-1)

5-bromo-2-methoxy-4-methyl-3-nitropyridine (100.15 g, 397.2 mmol) was added into a 2500 mL three-necked flask, DMF (500 mL) was added, the mixture was stirred for dissolution, and cooled to 0°C in an ice bath, and under nitrogen protection, sodium methoxide (10.9 g, 198.6 mmol) was added in batches, and then the mixture was heated to 90°C and was stirred for reaction for half an hour. After the temperature of the reaction solution was naturally cooled to below 80°C, N,N-dimethylformamide dimethyl acetal (386.4 g, 3.178 mol) was added in batches, the mixture was heated to 90°C, and the temperature was held for reaction for 1 hour. The reaction process was monitored by TCL (PE/EA=10/1), after the reaction was completed and the reaction solution was cooled to room temperature, the reaction solution was poured into ice water (1L), and a precipitated solid product was collected, washed with water (500 mL×3), and dried at 50°C under reduced pressure to obtain intermediate **Ia-1-1** (96.30 g, yield: 76.2%), ESI-MS m/z: 302/304 [M+H]⁺.

### Step 2: 4-bromo-7-methoxy-1H-pyrrolo[2,3-c]pyridine (la-1-2)

The above intermediate **Ia-1-1** (143.5 g, 466.5 mmol) was added into a 2000 mL three-necked flask, AcOH (400 mL) was added, the mixture was stirred fully, and cooled to 0°C in an ice bath, and under nitrogen protection, reduced iron powder (159.9 g, 2.798 mol) was added in batches, and then the mixture was heated to 110°C and was stirred for reaction for 4 hours. After the reaction was completed, the reaction solution was diluted by adding 800 mL of ethyl acetate, and washed and extracted with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1-2** (83.6 g, yield: 71.0%), ESI-MS m/z: 227/229 [M+H]⁺.

### Step 3: 4-bromo-7-methoxy-1-p-toluenesulfonyl-1H-pyrrolo[2,3-c]pyridine (la-1-3)

The above intermediate **Ia-1-2** (81.6 g, 352.3 mmol) was added into a 1500 mL three-necked flask, THF (500 mL) was added, the mixture was stirred fully, and cooled to 0°C in an ice bath, under nitrogen protection, NaH (60%, 28.20 g, 704.6 mmol) was added in batches, then the mixture was kept in ice bath for cooling and further stirred for reaction for 30 minutes, then tosyl chloride (76.18 g, 422.7 mmol) was added, and the mixture was naturally warmed to room temperature and stirred for reaction for 1 hour. The reaction process was monitored by TCL, after the reaction was completed, the reaction solution was cooled to room temperature, added with glacial ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1-3** (107.30 g, yield: 71.9%), ESI-MS m/z: 381/383 [M+H]⁺.

### Step 4: propyl 4-bromo-7-methoxy-1-p-toluenesulfonyl-1H-pyrrolo[2,3-c]pyridine-2-carboxylate (Ia-1-4)

The above intermediate **Ia-1-3** (106.2 g, 273.9 mmol) was added into a 1500 mL three-necked flask, THF (600 mL) was added and stirred for dissolution, the mixture was cooled to below -60°C in a dry-ice bath, under nitrogen protection, LDA in THF (2M, 208.0 mL, 410.8 mmol) was added dropwise in batches, then the mixture was kept in dry-ice bath for cooling and further stirred for reaction for 1 hour, then propyl chloroformate (51.35 g, 410.8 mmol) was added, and the mixture was stirred for reaction for 2 hours. The reaction process was monitored by TCL, after the reaction was completed, the solution was added dropwise with saturated ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1-4** (107.50 g, yield: 75.8%), ESI-MS m/z: 467/469 [M+H]⁺.

### Step 5: propyl 4-bromo-7-oxo-1-p-toluenesulfonyl-6,7-dihydro-1H-pyrrolo[2,3-c]pyridine-2-carboxylate (Ia-1-5)

The above intermediate **Ia-1-4** (104.2 g, 219.1 mmol) was added into a 1500 mL three-necked flask, acetonitrile solution (500 mL) was added and stirred for dissolution, the mixture was cooled to below -5°C in an ice bath, under nitrogen protection, NaI (50.3 g, 328.7 mmol) and Me₃Si-Cl (36.4 g, 328.7 mmol) were added in batches, and then the mixture was naturally warmed to room temperature, further stirred for reaction for 1 hour, and then heated to 60°C and stirred for reaction for 1 hour. The reaction process was monitored by TCL, after the reaction was completed, the solution was added with sodium thiosulfate solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **la-1-5** (86.9 g, yield: 79.0%), ESI-MS m/z: 453/455 [M+H]⁺.

### Step 6: propyl 4-bromo-6-methyl-7-oxo-1-p-toluenesulfonyl-6,7-dihydro-1H-pyrrolo[2,3-c]pyridine-2-carboxylate (Ia-1-6)

The above intermediate **la-1-5** (85.0 g, 184.3 mmol) was added into a 1500 mL three-necked flask, DMF (400 mL) was added and stirred for dissolution, Cs₂CO₃ (91.9 g, 276.4 mmol) was added at room temperature, the mixture was pre-stirred for 10 minutes and cooled to below 0°C in an ice bath, then iodomethane (29.36 g, 202.7 mmol) was added in batches, and the mixture was naturally warmed to room temperature and further stirred for reaction for 3 hours. The reaction process was monitored by TCL, after the reaction was completed, the solution was extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1-6** (70.8 g, yield: 74.2%), ESI-MS: *m*/*z* = 467/469 (M+H)⁺; ¹H NMR (400 MHz, *d₆*-DMSO) *δ* 8.29 (d, *J* = 8.4 Hz, 1H), 7.95 (m, 2H), 7.52 (d, *J* = 8.0 Hz, 2H), 7.04 (s, 1H), 4.31 (t, *J* = 6.4 Hz, 2H), 3.45 (s, 3H), 2.43 (s, 3H), 1.79 - 1.70 (m, 2H), 0.97 (t, *J* = 7.2 Hz, 3H).

### Step 7: 4-bromo-6-methyl-7-oxo-6,7-dihydro-1H-pyrrole[2,3-c]pyrimidine-2-carboxylic acid (Ia-1-7)

The above intermediate **Ia-1-6** (68.1 g, 143.2 mmol) was added into a 1500 mL three-necked flask, a mixed solution (500 mL) of methanol and water (3:1) was added and stirred for dissolution, LiOH (6.58 g, 358.1 mmol) was added at room temperature, and the mixture was further stirred at room temperature for 6 hours. The reaction process was monitored by TCL, after the reaction was completed, methanol was recovered, and the residue reaction solution was extracted with ethyl acetate and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1-7** (31.2 g, yield: 72.6%), ESI-MS: *m*/*z* = 271/273 (M+H)⁺.

### Step 8: 4-bromo-N-ethyl-6-methyl-7-oxo-6,7-dihydro-1H-pyrrolo[2,3-c]pyridine-2-carboxamide (Ic-1)

The above intermediate **Ia-1-7** (30.5 g, 110.7 mmol) was added into a 500 mL three-necked flask, acetonitrile solvent (180 mL) was added and stirred for dissolution, HOBt (30.2 g, 223.5 mmol), condensing agent EDCI (43.31 g, 221.4 mmol), and ethylamine solution were added at room temperature, and the mixture was stirred for reaction at room temperature for 8 hours. The reaction process was monitored by TCL, after the reaction was completed, the reaction solution was cooled to room temperature, added with water, and added with ethyl acetate for extraction, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ic-1** (23.8 g, yield: 65.4%), ESI-MS: *m*/*z* = 298/300 (M+H)⁺.

### Step 9: N-ethyl-6-methyl-7-oxo-4-(4,4,5,5-tetramethyl-1,3,2-dioxaborolan-2-yl)-6,7-dihydro-1H-pyrrolo[2,3-c]pyridine-2-carboxamide (Ia-1)

The above intermediate **Ic-1** (20.6 g, 67.90 mmol), bis(pinacolato)diboron (26.4 g, 101.9 mmol), ligand Xphos (1.65 g, 3.4 mmol), and potassium acetate (8.17 g, 81.5 mmol) were added into a 500 mL three-necked flask, 1,4-dioxane solvent (150 mL) was added, the mixture was stirred uniformly, the air was replaced with nitrogen three times, Pd₂(dba)₃ catalyst (3.15 g, 3.4 mmol) was added under a nitrogen flow, and then under nitrogen protection, the mixture was heated to 100°C for reaction for 3 hours. The reaction process was monitored by TCL, after the reaction was completed, ethyl acetate was added for dilution, the mixture was washed with water and extracted, washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **Ia-1** (14.7 g, yield: 56.4%), ESI-MS: *m*/*z*= 346.2 (M+H)⁺; ¹H NMR (400 MHz, DMSO-*d₆*) *δ* 12.12 (s, 1H), 9.39 (br-s, 1H), 7.60 (s, 1H), 7.05 (s, 1H), 3.51 (s, 3H), 3.29-3.25 (m, 2H), 1.31 (s, 12H), 1.13 (t, *J* = 3.2 Hz, 3H).

### Example 1

### Synthesis of N-ethyl-4-(5-(2-hydroxypropyl-2-yl)-3-(indolin-1-yl)thiophen-2-yl)-6-methyl-7-oxo-6,7-dihydro-1H-pyrrolo[2,3-c]pyridine-2-carboxamide (1)

### Synthetic Route:

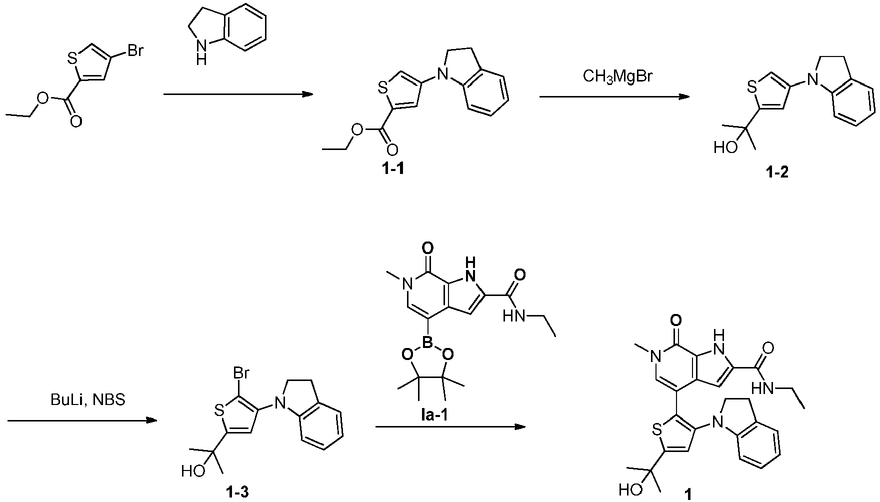

### Step 1: ethyl 4-(indolin-1-yl)thiophene-2-carboxylate (1-1)

Ethyl 4-bromothiophene-2-carboxylate (2.52 g, 10.55 mmol), indoline (1.92 g, 13.72 mmol), potassium acetate (1.26 g, 12.66 mmol), ligand Xantphos (187 mg, 0.3 mmol) and toluene (50 mL) were added into a 50 mL three-necked flask, the air was replaced with nitrogen three times, Pd₂(dba)₃ catalyst (295 mg, 0.3 mmol) was added under a nitrogen flow, and under nitrogen protection, the mixture was heated to 85°C for reaction for 2 hours. The reaction was monitored by TCL (PE/EA=10/1), after the reaction was completed, the reaction solution was cooled to room temperature, diluted with water, and extracted with ethyl acetate, the organic phases were combined, and washed with saturated sodium chloride solution, the organic layer was added with anhydrous sodium sulfate and stood overnight for drying, the desiccant was filtered out, and the filtrate was subjected to reduced pressure to recover the solvent, and purified via column chromatography, to obtain an off-white solid 1-1 (1.96 g, yield: 61.2%), ESI-MS m/z: 274.1(M+H)⁺.

### Step 2: 2-(4-(indolin-1-yl)thiophen-2-yl)propan-2-ol (1-2)

The intermediate **1-1** (1.92 g, 6.89 mmol) in the above step was added into a 50 mL three-necked flask, THF (30 mL) was added and stirred for dissolution, the mixture was cooled to below -5°C in an ice bath, and under nitrogen protection, a THF solution of methyl magnesium bromide (2M, 7.58 mL, 15.16 mmol) was added dropwise in batches, then the mixture was kept in the ice bath for cooling and further stirred for reaction for 2 hours, and the mixture was naturally warmed to room temperature and stirred for reaction for 2 hours. The reaction process was monitored by TCL, after the reaction was completed, the solution was added dropwise with saturated ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **1-2** (1.53 g, yield: 77.1%), ESI-MS m/z: 260.1 (M+H)⁺.

### Step 3: 2-(5-bromo-4-(indolin-1-yl)thiophen-2-yl)propan-2-ol (1-3)

The intermediate **1-2** (1.85 g, 7.00 mmol) in the above step was added into a 50 mL three-necked flask, THF (40 mL) was added and stirred for dissolution, the mixture was cooled to below -65°C in a dry-ice bath, under nitrogen protection, cyclohexane solution of n-butyllithium (2.5M, 6.2 mL, 15.4 mmol) was added dropwise in batches, then the mixture was kept in the dry-ice bath for cooling and further stirred for reaction for 1 hour, then N-bromosuccinimide (1.39 g, 7.8 mmol) was added, and the mixture was stirred for reaction for 3 hours. The reaction process was monitored by TCL, after the reaction was completed, the solution was added dropwise with saturated ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **1-3** (1.66 g, yield: 63.3%), ESI-MS m/z: 338/340 [M+H]⁺.

### Step 4: N-ethyl-4-(5-(2-hydroxypropyl-2-yl)-3-(indolin-1-yl)thiophen-2-yl)-6-methyl-7-oxo-6,7-dihydro-1H-pyrrolo[2,3-c]pyridine-2-carboxamide (1)

The intermediate 1-3 (0.53 g, 1.54 mmol) and the intermediate **Ia-1** (0.59 g, 1.70 mmol) in the above step, ligand Xphos (27 mg, 0.04 mmol), and cesium carbonate (0.77 g, 2.31 mmol) were added into a 50 mL three-necked flask, 1,4-dioxane solvent (15 mL) was added, the mixture was stirred uniformly, the air was replaced with nitrogen three times, Pd₂(dba)₃ catalyst (43.0 mg, 0.04 mmol) was added under a nitrogen flow, the air was replaced again with nitrogen two times, and under nitrogen protection, the mixture was heated to 105°C for reaction for 8 hours. The reaction process was monitored by TCL, after the reaction was completed, ethyl acetate was added for dilution, the mixture was washed with water and extracted, washed with saturated brine, the organic layer was added with anhydrous sodium sulfate and stood overnight for drying, the desiccant was filtered out, the filtrate was subjected to reduced pressure to recover the solvent and purified via flash chromatography, most of the organic solvent was recovered, and the product was freeze-dried to obtain 305 mg of the target compound **1 (yield: 40.9%),** ESI-MS: *m*/*z* = 477.2 (M+H)⁺; ¹H NMR (400 MHz, *d₆*-DMSO) *δ* 12.27 (s, -NH, 1H), 8.37 (s, -NH, 1H), 7.34 (s, 1H), 7.05 (s, 1H), 7.01 (m, 1H), 6.92 (s, 1H), 6.55 (m, 1H), 6.35 (m, 1H), 6.33 (m, 1H), 5.53 (s,-OH, 1H), 3.72 (t, *J* = 2.4 Hz, 2H), 3.34 (s, 3H), 3.24 (s, 2H), 2.96 (m, 2H), 1.53 (m, 6H), 1.13 (m, 3H).

### Example 2

### N-ethyl-4-(3-(7-fluoroindolin-1-yl)-5-(2-hydroxypropyl-2-yl)thiophen-2-yl)-6-methyl-7-oxo-6,7-dihydro-1H-pyrrolo[2,3-c]pyridine-2-carboxamide (2)

### Synthetic Route:

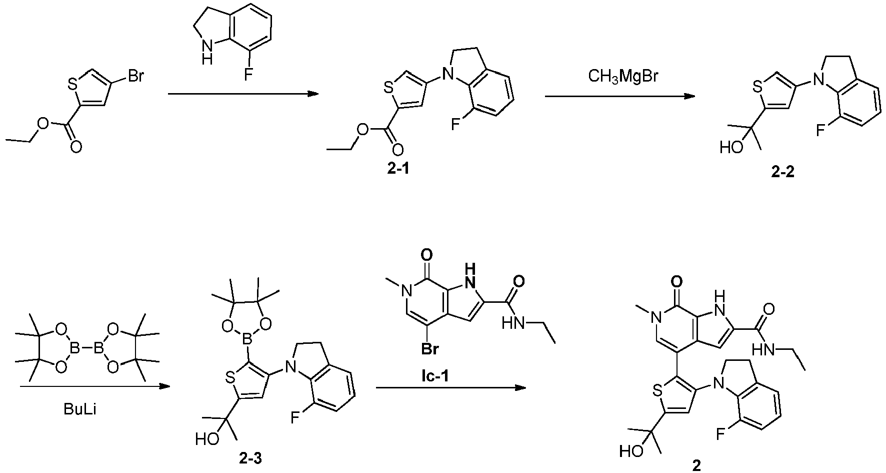

### Step 1: ethyl 4-(7-fluoro-indolin-1-yl)thiophene-2-carboxylate (2-1)

Ethyl 4-bromothiophene-2-carboxylate (2.0 g, 8.37 mmol), 7-fluoroindoline (1.75 g, 12.56 mmol), potassium acetate (1.0 g, 10.05 mmol), ligand Xantphos (148 mg, 0.25 mmol) and toluene (50 mL) were added into a 50 mL three-necked flask, the air was replaced with nitrogen three times, Pd₂(dba)₃ catalyst (295 mg, 0.25 mmol) was added under a nitrogen flow, and under nitrogen protection, the mixture was heated to 90°C for reaction for 1 hour. The reaction was monitored by TCL (PE/EA=10/1), after the reaction was completed, the reaction solution was cooled to room temperature, diluted with water, and extracted with ethyl acetate, the organic phases were combined, and washed with saturated sodium chloride solution, the organic layer was added with anhydrous sodium sulfate and stood overnight for drying, the desiccant was filtered out, and the filtrate was subjected to reduced pressure to recover the solvent, and purified via column chromatography, to obtain intermediate **2-1** (1.71 g, yield: 63.1%), ESI-MS m/z: 292.1(M+H)⁺.

### Step 2: 2-(4-(7-fluoro-indolin-1-yl)thiophen-2-yl)propan-2-ol (2-2)

The intermediate **2-1** (1.62 g, 5.45 mmol) in the above step was added into a 50 mL three-necked flask, THF (30 mL) was added and stirred for dissolution, the mixture was cooled to below -5°C in an ice bath, under nitrogen protection, a THF solution of methyl magnesium bromide (2M, 6.0 mL, 12.0 mmol) was added dropwise in batches, then the mixture was kept in the ice bath for cooling and further stirred for reaction for 2 hours, and the mixture was naturally warmed to room temperature and stirred for reaction for 2 hours. The reaction process was monitored by TCL, after the reaction was completed, the solution was added dropwise with saturated ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate 2-2 (1.24 g, yield: 73.8%), ESI-MS m/z: 278.1 (M+H)⁺.

### Step 3: 2-(4-(7-fluoro-indolin-1-yl)-5-(4,4,5,5-tetramethyl-1,3,2-dioxaborolan-2-yl)thiophene-2-yl)propan-2-ol (2-3)

The intermediate **2-2** (1.2 g, 4.24 mmol) in the above step was added into a 50 mL three-necked flask, THF (25 mL) was added and stirred for dissolution, the mixture was cooled to below -65°C in a dry-ice bath, under nitrogen protection, a cyclohexane solution of n-butyllithium (2.5M, 3.7 mL, 9.3 mmol) was added dropwise in batches, then the mixture was kept in the ice bath for cooling and further stirred for reaction for 1 hour, and the mixture was heated to -40°C and stirred for reaction for 2 hours. Under nitrogen protection, bis(pinacolato)diboron (1.31 g, 5.09 mmol) was added in batches, the reaction was continued for 1 hour, and the mixture was naturally warmed to room temperature and stirred for reaction for 3 hours. The reaction process was monitored by TCL, after the reaction was completed, the solution was added dropwise with saturated ammonium chloride solution for quenching the reaction, extracted with ethyl acetate, and washed with saturated brine, the organic layer was added with anhydrous sodium sulfate and stood overnight for drying, the desiccant was filtered out, and the filtrate was subjected to reduced pressure to recover the solvent, purified via column chromatography, and treated to obtain the intermediate **2-3** (1.0 g, yield: 53.8%), ESI-MS m/z: 404.2 (M+H)⁺.

### Step 4: N-ethyl-4-(3-(7-fluoroindolin-1-yl)-5-(2-hydroxypropyl-2-yl)thiophen-2-yl)-6-methyl-7-oxo-6,7-dihydro-1H-pyrrolo[2,3-c]pyridine-2-carboxamide (2)

The intermediate **2-3** (0.48 g, 1.17 mmol) and the intermediate **Ic-1** (0.53 g, 1.75 mmol) in the above step, ligand Xantphos (20.6 mg, 0.04 mmol), and cesium carbonate (0.58 g, 1.75 mmol) were added into a 50 mL three-necked flask, 1,4-dioxane solvent (15 mL) was added, the mixture was stirred uniformly, the air was replaced with nitrogen three times, Pd₂(dba)₃ catalyst (32.7 mg, 0.04 mmol) was added under a nitrogen flow, the air was replaced again with nitrogen two times, and under nitrogen protection, the mixture was heated to 105°C for reaction for 8 hours. The reaction process was monitored by TCL, after the reaction was completed, ethyl acetate was added for dilution, the mixture was washed with water and extracted, washed with saturated brine, the organic layer was added with anhydrous sodium sulfate and stood overnight for drying, the desiccant was filtered out, the filtrate was subjected to reduced pressure to recover the solvent and purified via flash chromatography, most of the organic solvent was recovered, and the product was freeze-dried to obtain 261 mg of the target compound **2 (yield: 44.4%),** ESI-MS: *m*/*z* = 495.2 (M+H)⁺; ¹H NMR (400 MHz, *d₆*-DMSO) *δ* 12.30(s, -NH, 1H), 8.44 (s, -NH, 1H), 7.28 (s, 1H), 7.24 (s, 1H), 7.20 (m, 1H), 7.13-7.16 (m, 1H), 6.89 (s, 1H), 6.60 (s, 1H), 4.89 (br-s,-OH, 1H), 4.01 (t, *J=* 2.4 Hz, 2H), 3.69 (s, 3H), 3.43-3.38 (m, 4H), 1.60 (m, 6H), 1.28 (m, 3H).

### Example 3

### N-ethyl-4-(5-(2-hydroxypropyl-2-yl)-3-(7-methylindolin-1-yl)thiophen-2-yl)-6-methyl-7-oxo-6,7-dihydro-1H-pyrrolo[2,3-c]pyridine-2-carboxamide (3)

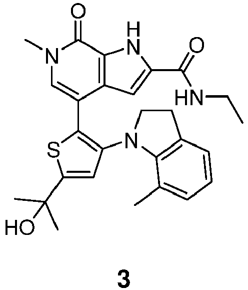

The target compound 3 was prepared by a method with reference to the preparation method in Example 1, except that 7-methylindoline was used instead of indoline, and the product was purified by the method described in Example 1. ESI-MS: *m*/*z* = 491.2 (M+H)⁺. ¹H NMR (400 MHz, d₆-DMSO) *δ* 12.32 (s, -NH, 1H), 8.42 (s, - NH, 1H), 7.32 (s, 1H), 6.95 (s, 1H), 6.79 (m, 1H), 6.68(m, 1H), 6.66(m, 1H), 6.64 (m, 1H), 5.05 (s,-OH, 1H), 3.58 (br-s, 2H), 3.56 (s, 3H), 3.20 (m, 2H), 2.96 (m, 2H), 1.86 (m, 3H), 1.50 (m, 6H), 1.15 (m, 3H).

### Example 4

### N-ethyl-4-(3-(5-fluoroindolin-1-yl)-5-(2-hydroxypropyl-2-yl)thiophen-2-yl)-6-methyl-7-oxo-6,7-dihydro-1H-pyrrolo[2,3-c]pyridine-2-carboxamide (4)

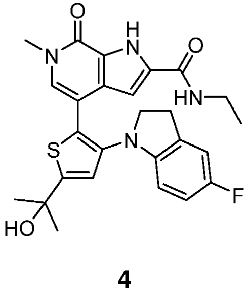

The target compound 4 was prepared by a method with reference to the preparation method in Example 1, except that 5-fluoroindoline was used instead of indoline, and the product was purified by the method described in Example 1. ESI-MS: *m*/*z*= 495.2 (M+H)⁺. ¹H NMR (400 MHz, *d₆*-DMSO) *δ* 12.35 (s, 1H), 8.45 (s, -NH, 1H), 7.72 (m, 1H), 7.28 (m, 1H), 6.98-7.02 (m, 2H), 6.74 (m, 1H), 6.32 (m, 1H), 5.61 (s, 1H), 3.82 (m, 2H), 3.57 (s, 3H), 3.34 (m, 2H), 3.03 (t, J = 3.5 Hz, 2H), 1.62 (s, 6H), 1.20 (t, J = 4.2 Hz, 3H).

### Example 5

### N-ethyl-4-(5-(2-hydroxypropyl-2-yl)-3-(5-trifluoromethylindolin-1-yl)thiophen-2-yl)-6-methyl-7-oxo-6,7-dihydro-1H-pyrrolo[2,3-c]pyridine-2-carboxamide (5)

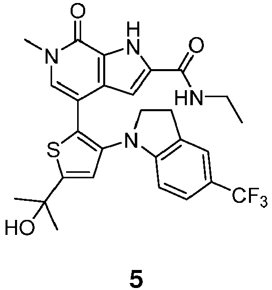

The target compound 5 was prepared by a method with reference to the preparation method in Example 1, except that 5-trifluoromethylindoline was used instead of indoline, and the product was purified by the method described in Example 1. ESI-MS: *m*/*z* = 545.2 (M+H)⁺.

### Example 6

### N-ethyl-4-(5-(2-hydroxypropyl-2-yl)-3-(6-fluoro-3,4-dihydroquinolin-1 (2H)-yl)thiophen-2-yl)-6-methyl-7-oxo-6,7-dihydro-1H-pyrrolo[2,3-c]pyridine-2-carboxamide (6)

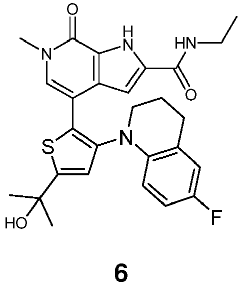

The target compound 6 was prepared by a method with reference to the preparation method in Example 1, except that 6-fluoro-3,4-dihydroquinoline was used instead of indoline, and the product was purified by the method described in Example 1. ESI-MS: *m*/*z* = 509.2 (M+H)⁺. ¹H NMR (400 MHz, *d₆*-DMSO) *δ* 12.28 (br-s, -NH, 1H), 8.36 (s, -NH, 1H), 7.25 (s, 1H), 6.95 (s, 1H), 6.86 (m, 1H), 6.78(m, 1H), 6.66(m, 1H), 6.33 (m, 1H), 5.56 (s,-OH, 1H), 3.44 (s, 3H), 3.25 (m, 4H), 2.68 (m, 2H), 1.80 (m, 2H), 1.52 (m, 6H) , 1.10 (m, 3H).

### Example 7

### N-ethyl-4-(5-(2-hydroxypropyl-2-yl)-3-(7-(trifluoromethyl)indolin-1-yl)thiophen-2-yl)-6-methyl-7-oxo-6,7-dihydro-1H-pyrrolo[2,3-c]pyridine-2-carboxamide (7)

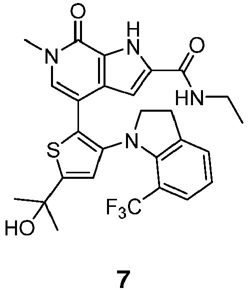

The target compound 7 was prepared by a method with reference to the preparation method in Example 1, except that 7-trifluoromethylindoline was used instead of indoline, and the product was purified by the method described in Example 1. ESI-MS: *m*/*z* = 545.2 (M+H)⁺; ¹H NMR (400 MHz, *d₆*-DMSO) δ 12.10(s, -NH, 1H), 8.64 (s, -NH, 1H), 7.48 (s, 1H), 7.34 (s, 1H), 7.25 (m, 1H), 7.19 (m, 1H), 6.82 (s, 1H), 6.63 (s, 1H), 4.86 (br-s,-OH, 1H), 4.03 (t, *J* = 2.4 Hz, 2H), 3.62 (s, 3H), 3.42 (m, 4H), 1.63 (m, 6H), 1.20 (m, 3H).

### Example 8

### 4-(3-(5-cyanoindolin-1-yl)-5-(2-hydroxypropyl-2-yl)thiophen-2-yl)-N-ethyl-6-methyl-7-oxo-6,7-dihydro-1H-pyrrolo[2,3-c]pyridine-2-carboxamide (8)

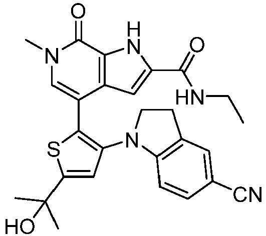

The target compound 8 was prepared by a method with reference to the preparation method in Example 1, except that 5-cyanoindoline was used instead of indoline, and the product was purified by the method described in Example 1. ESI-MS: *m*/*z* = 502.2 (M+H)⁺.

Other compounds 9 to 38 were prepared by the method shown in "General Reaction Scheme 1", and their mass spectrometry data is shown in the table below.

| Compound No. | Structural formula of the compound | Mass spectr um [M+1]⁺ | Compound No. | Structural formula of the compound | Mass spectrum [M+1]⁺ |
|---|---|---|---|---|---|
| 9 | 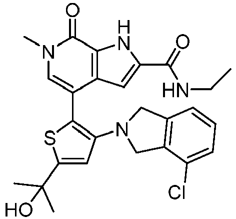 | 511.2 | 10 | 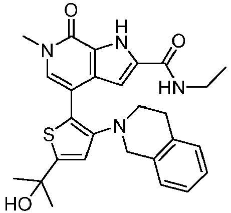 | 491.2 |
| 11 | 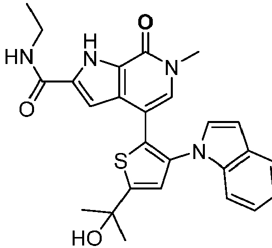 | 475.2 | 12 | 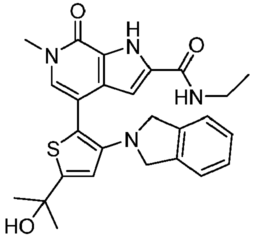 | 477.2 |
| 13 | 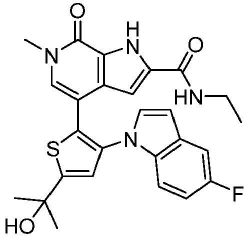 | 493.2 | 14 | 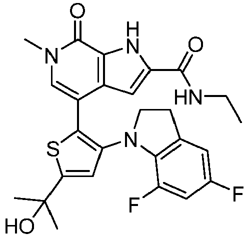 | 513.2 |
| 15 | 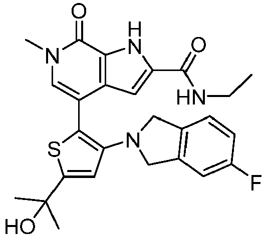 | 495.2 | 16 | 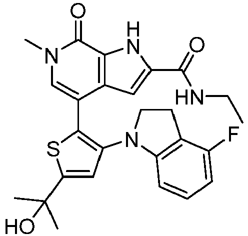 | 495.2 |
| 17 | 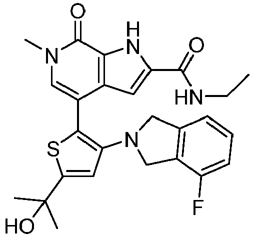 | 495.2 | 18 | 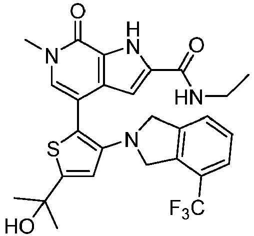 | 545.2 |
| 19 | 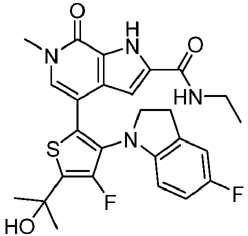 | 513.2 | 20 | 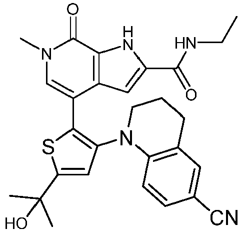 | 516.2 |
| 21 | 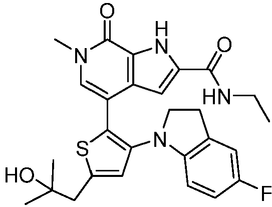 | 509.2 | 22 | 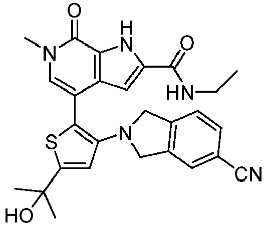 | 502.2 |
| 23 | 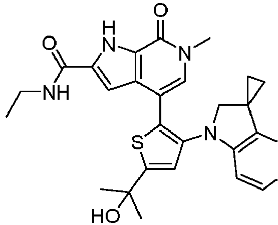 | 521.2 | 24 | 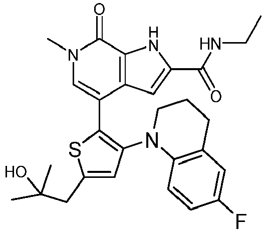 | 523.2 |
| 25 | 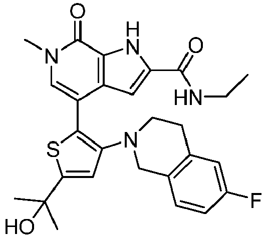 | 509.2 | 26 | 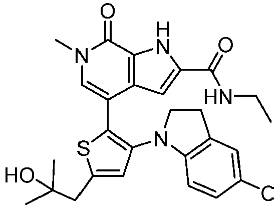 | 525.2 |
| 27 | 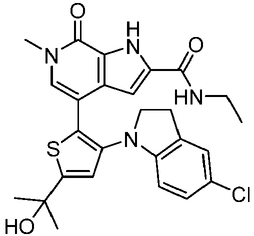 | 511.2 | 28 | 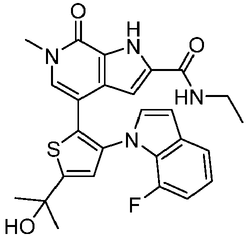 | 493.2 |
| 29 | 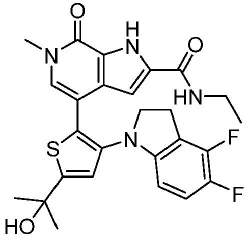 | 513.2 | 30 | 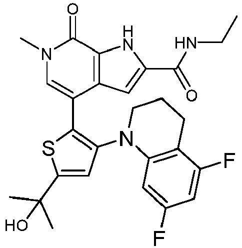 | 527.2 |
| 31 | 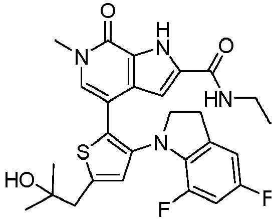 | 527.2 | 32 | 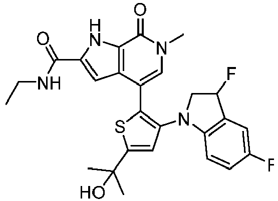 | 513.2 |
| 33 | 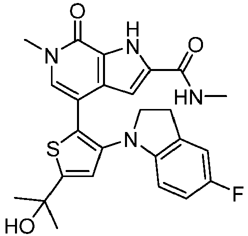 | 481.2 | 34 | 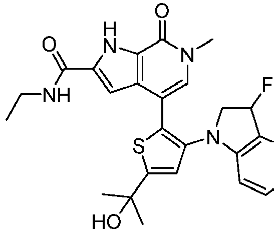 | 495.2 |
| 35 | 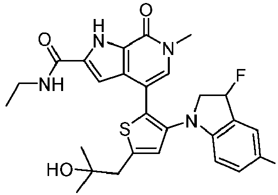 | 527.2 | 36 | 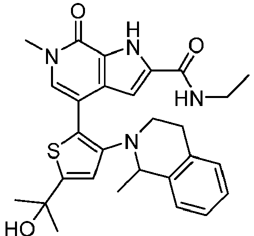 | 505.2 |
| 37 | 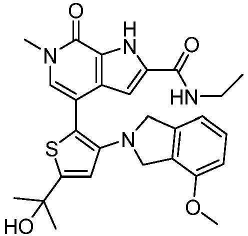 | 507.2 | 38 | 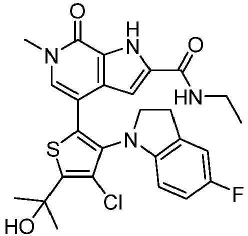 | 529.2 |

### Example 9 Cell Experiment (MV4-11 Cell Proliferation Experiment)

### Determination of Cell Viability with CCK-8 Reagent

### Day 1: Cell Plating

(1) MV4-11 cell suspension was pipetted from a culture flask into a 15 mL centrifuge tube, and the cell suspension was centrifuged at 1000 rpm for 4 minutes.
(2) A supernatant in the centrifuge tube was discarded, and an appropriate amount of fresh complete medium (IMDM+10%FBS+1%P/S) was added to resuspend the cells. 20 µL of cell suspension was collected, 20 µL of trypan blue was added, and CounterStar was used for counting.
(3) A required cell suspension volume and complete medium volume were calculated according to viable cell density and the required number of plated cells. MV4-11 cells were plated at a density of 1*10⁴ cells/well in 100 µL/well.
(4) 100 µL of the above cell suspension was pipetted into a 96-well plate by using an electric pipette. 150 µL of complete medium was added to blank control wells. PBS was added to peripheral wells of the 96-well plate, to avoid liquid evaporation. The 96-well plate was cultured overnight in an incubator.

### Day 2: Compound Preparation:

(1) The compound of the present application dissolved in DMSO and the positive control substance ABBV-744 were fetched, and subjected to serial dilution (dilution in column A, column B, ..., column J sequentially from left to right in a 96-well PCR plate).

An initial concentration of the solution in column A is 10 mM;
Column B: 2 µL of the solution in column A was diluted with 8 µL of DMSO to obtain a final concentration of 2000 µM;
Column C: 2 µL of the solution in column B was diluted with 8 µL of DMSO to obtain a final concentration of 400 µM;
Column D: 2 µL of the solution in column C was diluted with 8 µL of DMSO to obtain a final concentration of 80 µM;
Column E: 2 µL of the solution in column D was diluted with 8 µL of DMSO to obtain a final concentration of 16 µM;
Column F: 2 µL of the solution in column E was diluted with 8 µL of DMSO to obtain a final concentration of 3.2 µM;
Column G: 2 µL of the solution in column F was diluted with 8 µL of DMSO to obtain a final concentration of 0.64 µM;
Column H: 2 µL of the solution in column G was diluted with 8 µL of DMSO to obtain a final concentration of 0.128 µM;
Column I: 2 µL of the solution in column H was diluted with 8 µL of DMSO to obtain a final concentration of 0.0256 µM; and
Column J: 2 µL of the solution in column I was diluted with 8 µL of DMSO to obtain a final concentration of 0.00512 µM.

The suspension was centrifuged for 1 minute at 1000 rpm by using a low-speed centrifuge.
(2) 3×Compound Preparation: (diluted in a 96-well cell culture plate)

3 µL of each of the solutions in columns B to J was pipetted into 197 µL of complete medium by a 10 µL manual pipette to obtain 3×compound solutions with a final concentration of 30000, 6000, 1200, 240, 48, 9.6, 1.92, 0.384 and 0.0768 nM, respectively; and 3 µL of DMSO was pipetted into 197 µL of complete medium as a control. The resulting 96-well cell culture plate was put on an oscillator to be oscillated at 500 rpm for 30 minutes.

### Compound Treatment:

(1) The prepared 3×compound solutions with the above concentrations were each pipetted by a manual pipette with its volume set to 50 µL into a corresponding well of the 96-well plate after overnight culture on the first day, and the total culture volume of each well was 150 µL. The final concentrations of the compounds in the 96-well plate were 10 µM, 2 µM, 400 nM, 80 nM, 16 nM, 3.2 nM, 0.64 nM, 0.128 nM and 0.0256 nM, respectively. The resulting 96-well plate was put on an oscillator to be oscillated at 500 rpm for 10 minutes.
(2) The 96-well plate as prepared was put in a CO₂ incubator for 72-hour culture.

### Day 5: Detection:

(1) CCK-8 test solution was pipetted into the 96-well cell culture plate in an amount of 15 µL/well by a pipette with a volume set to 15 µL.
(2) The 96-well plate was put in the CO₂ incubator for 4-hour culture.
(3) The absorbance at 450 nm was read by an MD microplate reader, a suppression ratio was calculated, and the IC₅₀ value was calculated by Graph Pad.

Cell proliferation test results of the exemplary compounds of the present application are shown in Table 1.

**Table 1: Cell Proliferation Test Results of Exemplary Compounds**

| **Compound** | **MV4-11 cell IC₅₀ (nM)** | **Compound** | **MV4-11 cell IC₅₀ (nM)** |
|---|---|---|---|
| ABBV-744 | 7.22 | 4 | 6.30 |
| 1 | 1.98 | 5 | 12.13 |
| 2 | 24.21 | 6 | 8.20 |
| 3 | 39.81 | 7 | 17.31 |

It can be seen that the proliferation inhibition activity of the compounds of the present application for MV4-11 cells is equivalent to or even better than that of the positive control ABBV-744.

### Example 10 Pharmacokinetic Test of the Compound of the Present Application

SD rats were used as test animals. After gavage administration or tail vein injection of the compound of the present application to the rats, plasma drug concentrations were measured by the LC/MS/MS method at different moments, and pharmacokinetic features of the compound of the present application in the rats was studied.
Source of SD rats: Chengdu Dashuo Experimental Animal Co., Ltd.
Method of administration: single gavage administration and single tail vein injection
Dosage and concentration: 2 mg/kg rat body weight; 0.5 mg/mL
Formulation: 20% β-cyclodextrin solution
Sampling points: 5 min, 15 min, 30 min, 45 min, 1h, 1.5h, 2h, 4h, 5h, 6h, 7h, 8h, 9h and 24h

Standard curve preparation and sample treatment: 10 mg of the sample to be tested was accurately weighed and added into 1 mL of DMSO for dissolution, and the mixture was used as a stock solution. The stock solution was diluted with 50% (v/v) methanol aqueous solution to prepare standard working solutions having a concentration of 10, 20, 50, 100, 200, 500, 1000 and 2000 ng/mL, respectively. 5 µL of each of the standard working solutions was added to 45 µL of blank rat plasma to prepare standard samples containing the analyte at concentrations of 1.00, 2.00, 5.00, 10.00, 20.00, 50.00, 100.00 and 200.00 ng/mL, 200 µL of methanol (containing internal standard substance: 5ng/mL memantine hydrochloride) was added to each standard sample, the standard samples were oscillated for 3 minutes and centrifuged at 12000 rpm at 4°C for 10 minutes, and 100 mL of the supernatant was collected for LC-MS/MS analysis. The test results were calculated by Analyst^{®} software.

Preparation of quality control solution and sample treatment: 10 mg of the sample to be tested was accurately weighed and added into 1 mL of DMSO for dissolution, and the mixture was used as a stock solution. The stock solution was diluted with 50% (v/v) methanol aqueous solution to prepare quality control working solutions having a concentration of 30, 600 and 1600 ng/mL, respectively. 5 µL of each of the quality control working solutions was added to 45 µL of blank rat plasma to prepare quality control samples containing the analyte at concentrations of 3.00, 60.00 and 160.00 ng/mL, 200 µL of methanol (containing internal standard substance: 5ng/mL memantine hydrochloride) was added to each standard sample, the standard samples were oscillated for 3 minutes and centrifuged at 12000 rpm at 4°C for 10 minutes, and 100 mL of the supernatant was collected for LC-MS/MS analysis. The test results were calculated by Analyst^{®} software.

Pharmacokinetic parameters of the compound in exemplary Example 1 of the present application are shown in Table 2.

**Table 2: Pharmacokinetic Parameters of the Compound in Exemplary Example 1**

| Method of administration | Pharmacokinetic test (2 mg/kg) | | | | |
|---|---|---|---|---|---|
| | Time to peak | Peak concentration | Area under the curve | Half life | Retention time |
| | Tmax (h) | Cₘₐₓ (ng•mL⁻¹) | AUCₗₐₛₜ (h•ng•mL⁻¹) | T_{1/2} (h) | MRT (h) |
| Tail vein injection | 0.08 | 275.6 | 327.06 | 1.03 | 1.45 |
| Gavage | 0.29 | 143.00 | 249.70 | 0.83 | 1.37 |

The compound of the present application exhibits excellent pharmacokinetic property, and can be well absorbed via gavage administration, and a ratio of the curve area of gavage administration to that of tail vein injection (absolute bioavailability) is up to 76.35%.

### Example 11 In Vivo Efficacy Test of the Compound of the Present Application

Objective: To test the inhibitory effect of the test compound on the growth of xenograft tumor in nude mice with MV4-11 leukemia.

Method: NOD-SCID mice were subcutaneously inoculated with MV4-11 cells to establish an MV4-11 xenograft tumor model in mice. 16 days after inoculation (d16), average tumor volume was about 210 mm³. According to the tumor volumes, the tumor-bearing mice were grouped in a random block method, with 6 mice in each group. The groups included a solvent control group, an ABBV-744 control group, and a test sample group, the dosage was 5 mg/kg mouse body weight, and the dose volume was 10 mL/kg mouse body weight, once a day. Each group was gavage administered for 14 consecutive days, and the solvent control group was gavage administered with a blank solvent (20% β-cyclodextrin solution). Tumor volumes were measured twice weekly after administration of the test drug. The animals were euthanized after the test.

In vivo efficacy results of the compound in exemplary Example 1 of the present application are shown in Table 3.

**Table 3: In Vivo Efficacy Results of Each Group of Animals**

| Group | Number of animals (pcs) | Days of administration (days) | Relative tumor proliferation rate T/C (%) | Dosage and frequency |
|---|---|---|---|---|
| Solvent control group | 6 | 14 | - | - |
| ABBV-744 group | 6 | 14 | 45.98 | 5 mg/kg, qd |
| Test sample group (Example 1) | 6 | 14 | 33.15 | 5 mg/kg, qd |

Compared with the solvent control group and the ABBV-744 control group, the test group administered with the compound of the present application (test sample group) shows significant in vivo tumor inhibition activity (P<0.01), and the in vivo tumor inhibition activity of the compound of the present application is better than that in the ABBV-744 control group.

### Example 12 hERG Inhibition Test of the Compound of the Present Application

An hERG current was recorded by the whole-cell patch clamp technique. A suspension of HEK293 cells stably expressing the hERG ion channel was added to a 35 mm culture dish, and the culture dish was put on a stage of an inverted microscope. After the cells adhered to the wall, the extracellular fluid was used for perfusion (10 mM HEPES, 145 mM NaCl, 4.0 mM KCI, 2.0 mM CaCl₂, 1.0 mM MgCl₂, and 10 mM Glucose were adjusted with sodium hydroxide until the pH reached 7.3 to 7.4 and the osmotic pressure reached 290 to 310 mOsm, and the mixture was filtered and stored at 4°C) at a flow rate of 1.0-2.0 mL/min. The glass microelectrode was formed by pulling by a microelectrode puller in two steps, and after being filled with an inner solution of the electrode (120 mM KCI, 31.25 mM KOH, 5.374 mM CaCl₂, 1.75 mM MgCl₂, 10 mM EGTA, 10 mM HEPES and 4.0 mM Na2-ATP adjusted with potassium hydroxide until the pH reached 7.2 to 7.3 and the osmotic pressure reached 290 to 310 mOsm, and the mixture was filtered, then subpackaged and stored at -20°C), the glass microelectrode had a resistance value of 2-5 MΩ in water. After the whole-cell recording mode was established, clamping potential was maintained at -80 mV, a depolarization voltage was applied to reach a voltage of +60 mV and kept for 850 ms, and then repolarization was performed to reach a voltage of -50 mV and kept for 1275 ms to elicit an hERG tail current. The pulse program was repeated every 15 seconds throughout the whole test. After the current became stable, the administration method of continuous extracellular perfusion from low concentration to high concentration was used, and the perfusion started from low concentration and continued until the drug effect was stable, and then the perfusion of the next concentration was performed.

After the test, stimulus release and signal acquisition were carried out through PatchMaster software. Graphpad Prism 8.0 was used for further data analysis and curve fitting. Effects of each compound at different concentrations were represented with an inhibition rate of the tail current. Inhibition rate = (peak tail current before administration - peak tail current after administration)/peak tail current before administration × 100%. Half-maximal inhibitory concentration IC₅₀ of the test samples was obtained by the fitting analysis with Hill equation.

**Table 4: Test Results of the Inhibitory Effect of the Compound in Exemplary Example 1 on hERG**

| Compound | Concent ration (µM) | hERG Inhibition rate (%) | | Average hERG inhibition rate (%) | SD |
|---|---|---|---|---|---|
| | | Cell 1 | Cell 2 | | |
| Example 1 | 0.37 | 0.98 | 6.79 | 3.89 | 4.11 |
| | 1.11 | -0.28 | 8.97 | 4.35 | 6.54 |
| | 3.33 | 2.97 | 17.30 | 10.13 | 10.13 |
| | 10.00 | 14.56 | 28.55 | 21.56 | 9.89 |
| | 30.00 | 54.34 | 58.29 | 56.32 | 2.79 |
| ABBV744 | 0.37 | 8.24 | 2.46 | 5.35 | 4.09 |
| | 1.11 | 23.01 | 11.75 | 17.38 | 7.96 |
| | 3.33 | 57.73 | 51.66 | 54.70 | 4.29 |
| | 10.00 | 87.92 | 87.33 | 87.62 | 0.42 |
| | 30.00 | 99.44 | 98.68 | 99.06 | 0.54 |

IC₅₀ of the compound in Example 1 on hERG was 26.771 µM, IC₅₀ of the control compound ABBV-744 on hERG was 3.117 µM, and therefore, compared with the control compound ABBV-744, the compound of the present application had a lower risk of hERG cardiotoxicity (P<0.05).

It should be understood that the above detailed descriptions and accompanying examples are exemplary only, and should not be construed as a limitation on the scope of the present application that is defined only by the appended claims and their equivalents. Persons skilled in the art can easily understand various changes and modifications made to the disclosed embodiments. Such changes and modifications may be made without departing from the spirit and scope of the present application, and include but are not limited to those changes and modifications related to the chemical structures, substituents, derivatives, intermediates, syntheses, formulations and/or use methods in the present application. These modifications, changes or recombinations of different embodiments shall all fall within the protection scope of the present application. All publications, patents, and patent applications cited herein are hereby incorporated by reference in their entirety for all purposes.

## Claims

1. A compound of formula (I) or a pharmaceutically acceptable salt thereof: 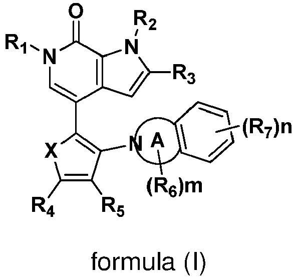 wherein:
ring A is a 5- to 8-membered saturated or unsaturated N-containing heterocycle, and together with its adjacent benzene ring forms a fused ring;
X is O or S;
m is 0, 1, 2, 3, 4, 5 or 6;
n is 0, 1, 2, 3 or 4;
R₁ is hydrogen or a C₁-C₆ alkyl group;
R₂ is hydrogen or a C₁-C₆ alkyl group or a C₃-C₆ cycloalkyl group;
R₃ is hydrogen, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
R₄ is hydrogen, -C(CH₃)₂OH, -CH₂C(CH₃)₂OH, -C(O)Rₓ, -C(O)ORₓ, -C(O)NHRₓ, -C(O)NRₓ₁R_{y1}, -S(O)₂Rₓ, -S(O)₂NRₓ₁R_{y1} or -N(Rₓ)S(O)₂R_{y};
R₅ is hydrogen, halogen, a cyano group, a hydroxyl group, an amino group, a nitro group, a C₁-C₆ alkyl group, a C₃-C₆ cycloalkyl group, a C₁-C₃ alkoxy group or a C₁-C₃ haloalkyl group;
R₆ is hydrogen, halogen, a cyano group, a hydroxyl group, an amino group, a C₁-C₆ alkyl group, a C₃-C₆ cycloalkyl group, a C₁-C₃ alkoxy group or a C₁-C₃ haloalkyl group, or two R₆ groups on the same carbon atom can form a C₃-C₈ spiro group together;
R₇ is hydrogen, halogen, a cyano group, a hydroxyl group, an amino group, a nitro group, a C₁-C₆ alkyl group, a C₃-C₆ cycloalkyl group, a C₁-C₃ alkoxy group, a C₁-C₃ haloalkyl group, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
Rₓ and R_{y} are each independently selected from hydrogen, a C₁-C₆ alkyl group or a C₃-C₆ cycloalkyl group; and
Rₓ₁ and R_{y1} are each independently selected from hydrogen, a C₁-C₆ alkyl group or a C₁-C₆ heteroalkyl group, or Rₓ₁, R_{y1} and a nitrogen atom are connected to form a 3- to 8-membered ring.

2. The compound of formula (I) or a pharmaceutically acceptable salt thereof according to claim 1, wherein
ring A is a 5- to 6-membered saturated or unsaturated N-containing heterocycle; and
preferably, the fused ring formed by ring A and its adjacent benzene ring is selected from: 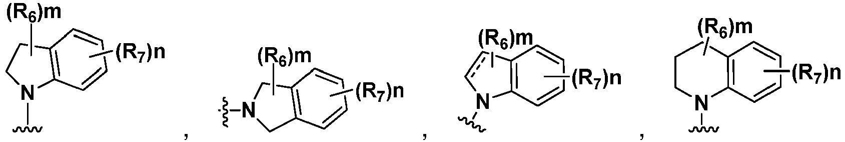 or

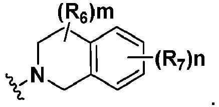

3. The compound of formula (I) or a pharmaceutically acceptable salt thereof according to claim 1 or 2, wherein, R₁ is hydrogen or a C₁-C₄ alkyl group, preferably, hydrogen or a C₁-C₃ alkyl group, and more preferably, hydrogen, a methyl group, an ethyl group, a n-propyl group or an isopropyl group.

4. The compound of formula (I) or a pharmaceutically acceptable salt thereof according to any one of claims 1 to 3, wherein R₂ is hydrogen or a C₁-C₃ alkyl group, preferably, hydrogen, a methyl group, an ethyl group, a n-propyl group, an isopropyl group or a cyclopropyl group.

5. The compound of formula (I) or a pharmaceutically acceptable salt thereof according to any one of claims 1 to 4, wherein,
R₃ is hydrogen, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1}, Rₓ and R_{y} are each independently selected from hydrogen, a methyl group, an ethyl group, an isopropyl group or a cyclopropyl group, and Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring: 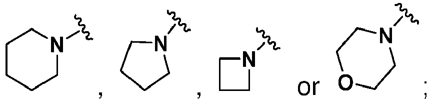
preferably, R₃ is hydrogen, -C(O)ORₓ, -C(O)NHRₓ or -C(O)NRₓ₁R_{y1}, Rₓ is selected from hydrogen or a C₁-C₆ alkyl group, and Rₓ₁ and R_{y1} are each independently selected from hydrogen or a C₁-C₆ alkyl group; or
preferably, R₃ is hydrogen, -C(O)Rₓ, -C(O)ORₓ, -C(O)NHRₓ, -C(O)NRₓ₁R_{y1}, - S(O)₂NRₓ₁R_{y1}, -N(Rₓ)S(O)₂R_{y} or -NRₓ₁R_{y1}, Rₓ and R_{y} are each independently selected from hydrogen, a methyl group, an ethyl group, an isopropyl group or a cyclopropyl group, and Rₓ₁ and R_{y1} are each independently selected from hydrogen, a methyl group, an ethyl group, an isopropyl group or a cyclopropyl group.

6. The compound of formula (I) or a pharmaceutically acceptable salt thereof according to any one of claims 1 to 5, wherein
R₄ is hydrogen, -C(CH₃)₂OH, -CH₂C(CH₃)₂OH, -C(O)Rₓ, -C(O)ORₓ, -C(O)NHRₓ, -C(O)NRₓ₁R_{y1}, -S(O)₂Rₓ, -S(O)₂NRₓ₁R_{y1} or -N(Rₓ)S(O)₂R_{y}, Rₓ and R_{y} are each independently selected from hydrogen, a methyl group, an ethyl group, an isopropyl group or a cyclopropyl group, and Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring: 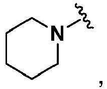 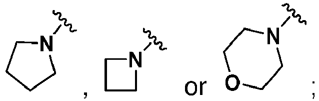
preferably, R₄ is hydrogen, -C(CH₃)₂OH, -CH₂C(CH₃)₂OH, -C(O)ORₓ or - C(O)NHRₓ, and Rₓ is hydrogen or a methyl group; or
preferably, R₄ is hydrogen, -C(CH₃)₂OH, -CH₂C(CH₃)₂OH, -C(O)Rₓ, -C(O)ORₓ, - C(O)NHRₓ, -C(O)NRₓ₁R_{y1}, -S(O)₂Rₓ, -S(O)₂NRₓ₁R_{y1} or -N(Rₓ)S(O)₂R_{y}, Rₓ and R_{y} are each independently selected from hydrogen, a methyl group, an ethyl group, an isopropyl group or a cyclopropyl group, and Rₓ₁ and R_{y1} are each independently selected from hydrogen, a methyl group, an ethyl group, an isopropyl group or a cyclopropyl group.

7. The compound of formula (I) or a pharmaceutically acceptable salt thereof according to any one of claims 1 to 6, wherein R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, preferably hydrogen, a hydroxyl group, an amino group, a methyl group or a methoxy group.

8. The compound of formula (I) or a pharmaceutically acceptable salt thereof according to any one of claims 1 to 7, wherein
R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃-C₆ spiro group;
preferably, R₆ is hydrogen, halogen, a hydroxyl group, an amino group, a C₁-C₄ alkyl group, a C₃-C₄ cycloalkyl group or a C₁-C₃ alkoxy group, or two R₆ groups on the same carbon atom form a C₃-C₄ spiro group; and
more preferably, R₆ is hydrogen, halogen, a hydroxyl group, an amino group, a methyl group, a cyclopropyl group or a methoxy group, or two R₆ groups on the same carbon atom form a C₃ spiro group.

9. The compound of formula (I) or a pharmaceutically acceptable salt thereof according to any one of claims 1 to 8, wherein
R₇ is hydrogen, halogen, a cyano group, a hydroxyl group, an amino group, a nitro group, a C₁-C₆ alkyl group, a C₃-C₆ cycloalkyl group, a C₁-C₃ alkoxy group, a C₁-C₃ haloalkyl group, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1}, preferably, is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group, a trifluoromethyl group, -C(O)Rₓ, -C(O)ORₓ, -C(O)NHRₓ, -C(O)NRₓ₁R_{y1}, - S(O)₂NRₓ₁R_{y1}, -N(Rₓ)S(O)₂R_{y} or -NRₓ₁R_{y1};
Rₓ and R_{y} are each independently selected from hydrogen, a methyl group, an ethyl group, an isopropyl group or a cyclopropyl group; and
Rₓ₁ and R_{y1} are each independently hydrogen, a methyl group, an ethyl group, an isopropyl group or a cyclopropyl group, or Rₓ₁, R_{y1} and a nitrogen atom are connected to form the following ring: 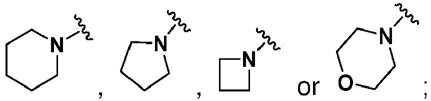
preferably, R₇ is hydrogen, halogen, a cyano group, a hydroxyl group, an amino group, a C₁-C₄ alkyl group, a C₁-C₃ alkoxy group, a C₁-C₃ haloalkyl group, -C(O)Rₓ, - C(O)ORₓ, -C(O)NHRₓ or -C(O)NRₓ₁R_{y1}, Rₓ is hydrogen, a methyl group or an ethyl group, and Rₓ₁ and R_{y1} are independently hydrogen or a methyl group;
preferably, R₇ is hydrogen, halogen, a cyano group, a hydroxyl group, an amino group, a C₁-C₄ alkyl group, a C₁-C₃ alkoxy group or a C₁-C₃ haloalkyl group; or
preferably, R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group or a trifluoromethyl group.

10. The compound of formula (I) or a pharmaceutically acceptable salt thereof according to any one of claims 1 to 9, wherein X is S.

11. The compound of formula (I) or a pharmaceutically acceptable salt thereof according to claim 1, wherein,
R₁ is hydrogen, a methyl group, an ethyl group, a n-propyl group or an isopropyl group;
R₂ is hydrogen, a methyl group, an ethyl group, a n-propyl group, an isopropyl group or a cyclopropyl group;
R₃ is hydrogen, -C(O)Rₓ, -C(O)ORₓ, -C(O)NHRₓ, -C(O)NRₓ₁R_{y1}, -S(O)₂NRₓ₁R_{y1}, - N(Rₓ)S(O)₂R_{y} or -NRₓ₁R_{y1};
R₄ is hydrogen, -C(CH₃)₂OH, -CH₂C(CH₃)₂OH, -C(O)Rₓ, -C(O)ORₓ, -C(O)NHRₓ, -C(O)NRₓ₁R_{y1}, -S(O)₂Rₓ, -S(O)₂NRₓ₁R_{y1} or -N(Rₓ)S(O)₂R_{y};
R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group;
R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃-C₆ spiro group;
R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group, a trifluoromethyl group, -C(O)Rₓ, -C(O)ORₓ, -C(O)NHRₓ, -C(O)NRₓ₁R_{y1}, -S(O)₂NRₓ₁R_{y1}, -N(Rₓ)S(O)₂R_{y} or - NRₓ₁R_{y};
Rₓ and R_{y} are each independently selected from hydrogen, a methyl group, an ethyl group, an isopropyl group or a cyclopropyl group; and
Rₓ₁ and R_{y1} are each independently selected from hydrogen, a methyl group, an ethyl group, an isopropyl group or a cyclopropyl group, or Rₓ₁, R_{y1} and a nitrogen atom are connected to form the following ring: 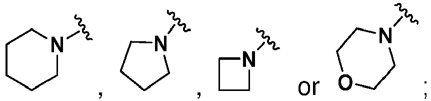
m is 0, 1, 2, 3, 4, 5 or 6; and
n is 0, 1, 2, 3 or 4;
or
ring A is a 5- to 6-membered saturated or unsaturated N-containing heterocycle;
X is O or S;
R₁ is hydrogen or a C₁-C₄ alkyl group;
R₂ is hydrogen or a C₁-C₃ alkyl group;
R₃ is hydrogen, -C(O)ORₓ, -C(O)NHRₓ or -C(O)NRₓ₁R_{y1};
R₄ is hydrogen, -C(CH₃)₂OH, -CH₂C(CH₃)₂OH, -C(O)ORₓ or -C(O)NHRₓ;
R₅ is hydrogen, a hydroxyl group, an amino group, a methyl group or a methoxy group;
R₆ is hydrogen, halogen, a hydroxyl group, an amino group, a C₁-C₄ alkyl group, a C₃-C₄ cycloalkyl group or a C₁-C₃ alkoxy group, or two R₆ groups on the same carbon atom form a C₃-C₄ spiro group;
R₇ is hydrogen, halogen, a cyano group, a hydroxyl group, an amino group, a C₁-C₄ alkyl group, a C₁-C₃ alkoxy group, a C₁-C₃ haloalkyl group, -C(O)Rₓ, -C(O)ORₓ, - C(O)NHRₓ or -C(O)NRₓ₁R_{y1};
Rₓ is selected from hydrogen or a C₁-C₆ alkyl group;
Rₓ₁ and R_{y1} are each independently selected from hydrogen or a C₁-C₆ alkyl group;
m is 0, 1, 2 or 3; and
n is 0, 1, 2 or 3;
preferably,
X is O or S;
R₁ is hydrogen, a methyl group, an ethyl group, a n-propyl group or an isopropyl group;
R₂ is hydrogen, a methyl group, an ethyl group, a n-propyl group, an isopropyl group or a cyclopropyl group;
R₃ is -C(O)NHRₓ;
R₄ is -CH₂C(CH₃)₂OH, -C(CH₃)₂OH or -C(O)ORₓ;
Rₓ is selected from hydrogen, a methyl group, an ethyl group, an isopropyl group or a cyclopropyl group;
R₅ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group;
R₆ is hydrogen, fluorine, chlorine, bromine, iodine, a cyano group, a hydroxyl group, an amino group, a methyl group, an ethyl group, an isopropyl group, a cyclopropyl group, a methoxy group, an ethoxy group or a trifluoromethyl group, or two R₆ groups on the same carbon atom form a C₃ spiro group;
R₇ is hydrogen, fluorine, chlorine, bromine, iodine, a cyano group, a hydroxyl group, an amino group, a nitro group, a methyl group, an ethyl group, an isopropyl group, a cyclopropyl group, a methoxy group, an ethoxy group or a trifluoromethyl group;
m is 0, 1 or 2; and
n is 0, 1 or 2;
preferably,
Xis S;
R₁ is a methyl group;
R₂ is hydrogen or a cyclopropyl group;
R₃ is -C(O)NHCH₃ or -C(O)NHCH₂CH₃;
R₄ is -CH₂C(CH₃)₂OH, -C(O)OCH₂CH₃ or -C(CH₃)₂OH;
R₅ is hydrogen, fluorine, chlorine, bromine, iodine or a methyl group;
R₆ is hydrogen, fluorine, chlorine, bromine, iodine, a methyl group, an ethyl group, an isopropyl group or a cyclopropyl group, or two R₆ groups on the same carbon atom form a C₃ spiro group;
R₇ is hydrogen, fluorine, chlorine, bromine, iodine, a cyano group, a methyl group, an ethyl group, an isopropyl group, a cyclopropyl group, a methoxy group, an ethoxy group or a trifluoromethyl group;
m is 0, 1 or 2; and
n is 0, 1 or 2; and
more preferably,
ring A is a 5- to 6-membered saturated or unsaturated N-containing heterocycle; and
Xis S;
R₁ is a methyl group;
R₂ is hydrogen or a cyclopropyl group;
R₃ is -C(O)NHCH₃ or -C(O)NHCH₂CH₃;
R₄ is -CH₂C(CH₃)₂OH, -C(O)OCH₂CH₃ or -C(CH₃)₂OH;
R₅ is hydrogen, fluorine, chlorine, bromine, iodine or a methyl group;
R₆ is hydrogen, fluorine, chlorine, bromine, iodine, a methyl group, an ethyl group, an isopropyl group or a cyclopropyl group, or two R₆ groups on the same carbon atom form a C₃ spiro group;
R₇ is hydrogen, fluorine, chlorine, bromine, iodine, a cyano group, a methyl group, an ethyl group, an isopropyl group, a cyclopropyl group, a methoxy group, an ethoxy group or a trifluoromethyl group;
m is 0, 1 or 2; and
n is 0, 1 or 2;
more preferably,
Xis S;
R₁ is a methyl group;
R₂ is hydrogen;
R₃ is -C(O)NHCH₂CH₃;
R₄ is -C(CH₃)₂OH;
R₅ is hydrogen;
R₆ is hydrogen;
R₇ is hydrogen, fluorine, a cyano group, a methyl group or a trifluoromethyl group;
m is 0 or 1; and
n is 0 or 1; and
even more preferably,
Xis S;
R₁ is a methyl group;
R₂ is hydrogen;
R₃ is -C(O)NHCH₂CH₃;
R₄ is -C(CH₃)₂OH;
R₅ is hydrogen;
R₆ is hydrogen;
R₇ is hydrogen or fluorine;
m is 0 or 1; and
n is 0 or 1.

12. The compound of formula (I) or a pharmaceutically acceptable salt thereof according to any one of claims 1 to 11, wherein the compound is selected from:

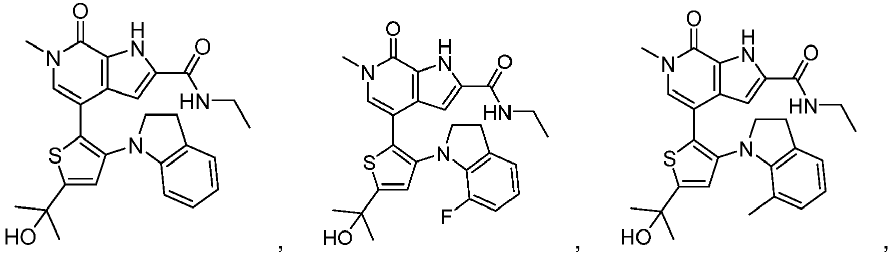

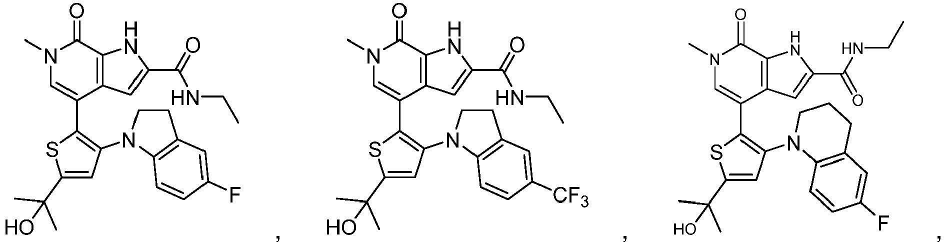

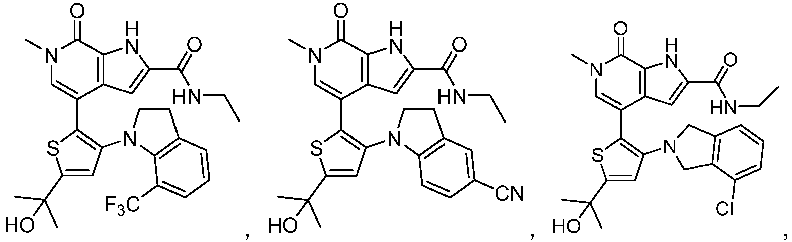

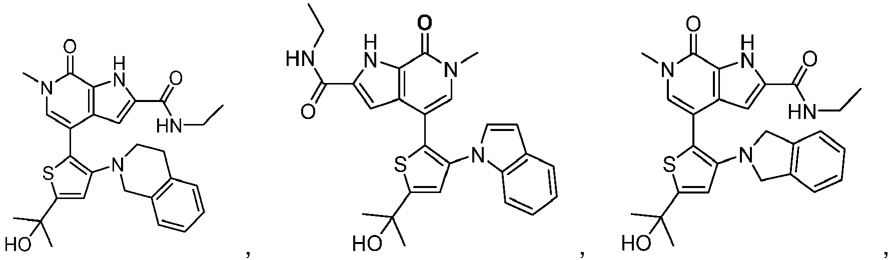

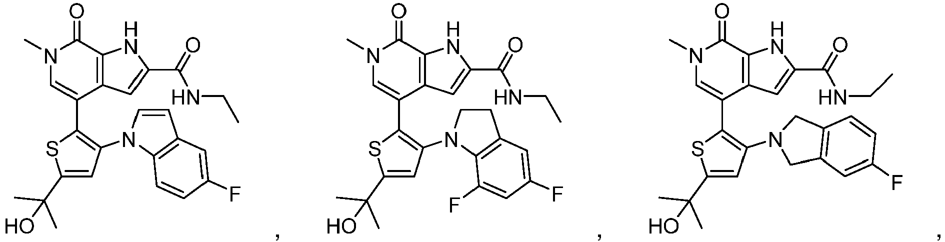

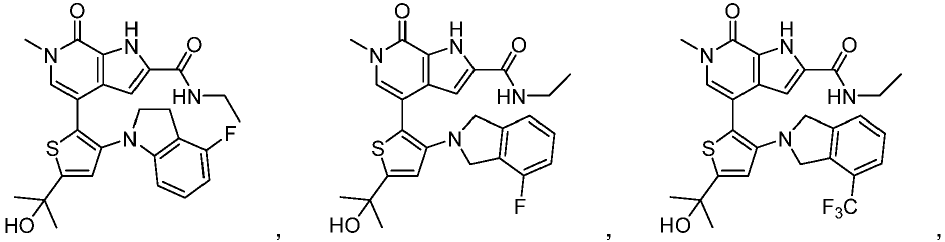

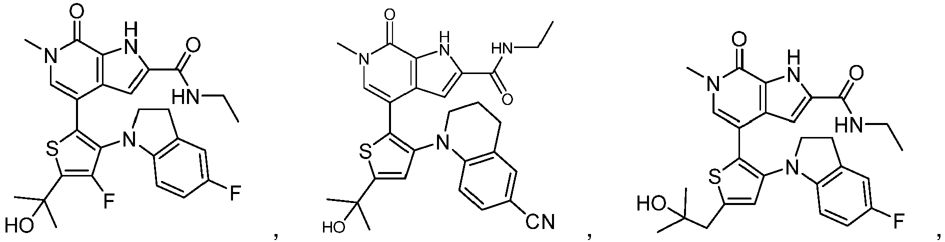

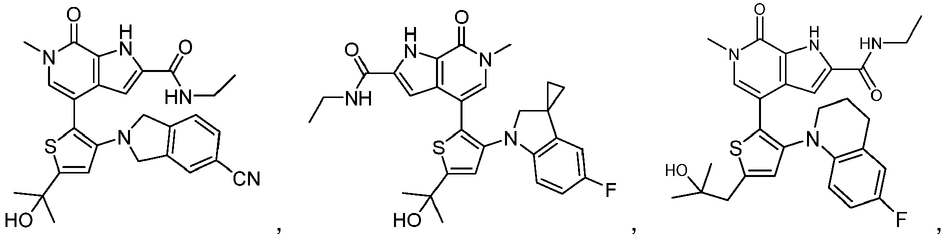

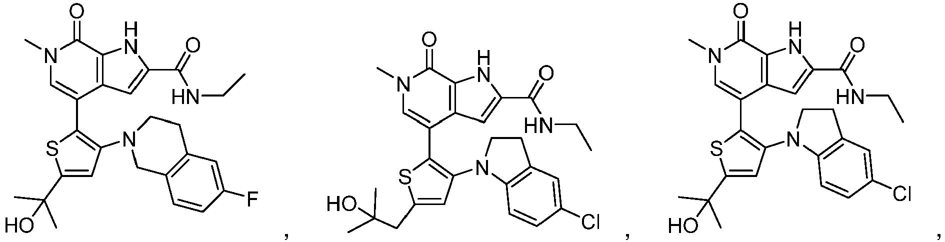

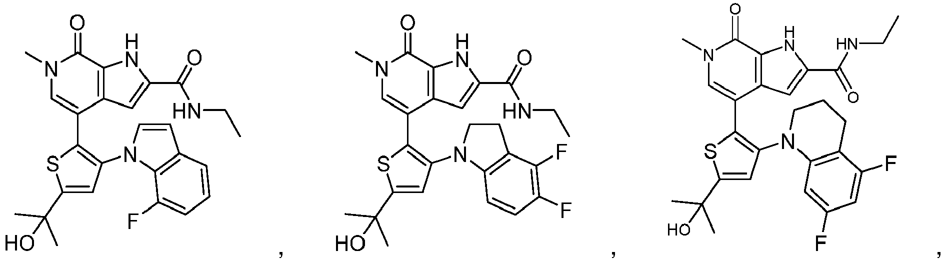

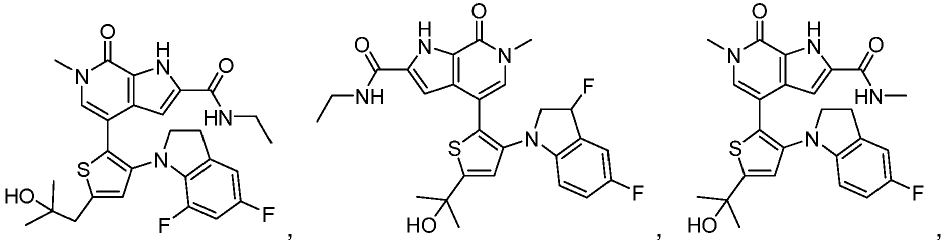

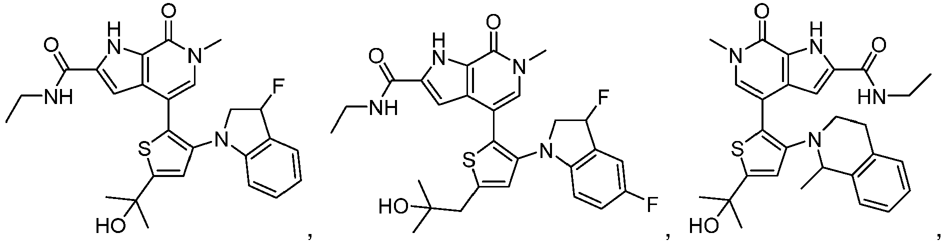

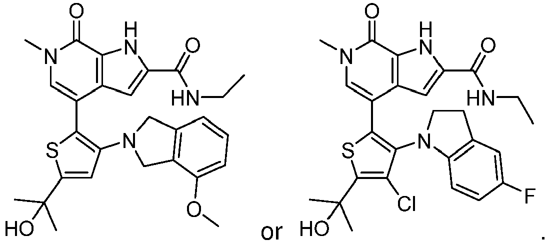

13. A method for synthesizing the compound of formula (I) or a pharmaceutically acceptable salt thereof according to any one of claims 1 to 12, comprising: 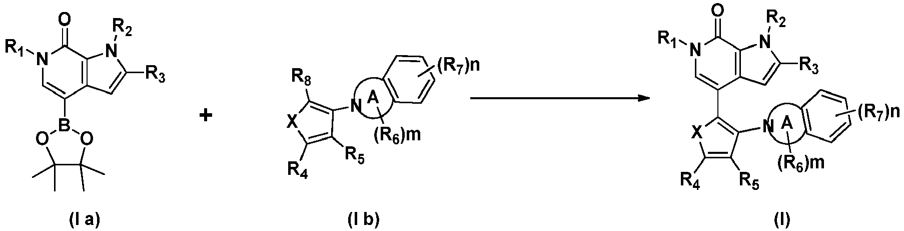 , or 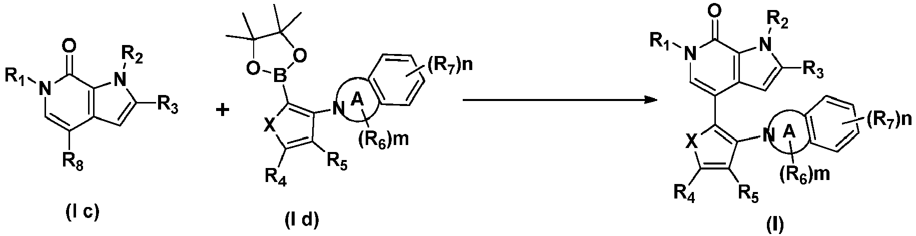
subjecting a compound of formula (Ia) and a compound of formula (Ib), or a compound of formula (Ic) and a compound of formula (Id) to a catalytic coupling reaction to obtain a compound of formula (I), wherein each of the groups is defined according to any one of claims 1 to 12, and R₈ is selected from Cl, Br or I, wherein
preferably, the catalytic coupling reaction is carried out in 1,4-dioxane in the presence of a ligand, an inorganic base and a palladium catalyst; and
preferably, the catalytic coupling reaction is carried out in an inert atmosphere at a temperature of 80°C to 120°C for 6 to 10 hours.

14. An intermediate, wherein the intermediate has a structure represented by formula (Ib) or formula (Id) below: 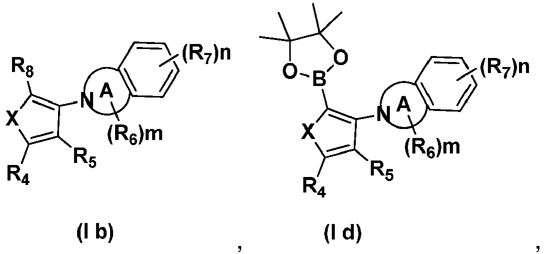 wherein, each of the groups is defined according to any one of claims 1 to 12, and R₈ is selected from Cl, Br or I.

15. A pharmaceutical composition, comprising the compound of formula (I) or a pharmaceutically acceptable salt thereof according to any one of claims 1 to 12.

16. A compound of formula (I) or a pharmaceutically acceptable salt thereof according to any one of claims 1 to 12, or a pharmaceutical composition according to claim 15 for use in prevention and/or treatment of a BET protein-associated disease;
preferably, the BET protein-associated disease is selected from a tumor disease, an inflammatory disease, an autoimmune disease and a viral infection; and
preferably, the tumor disease comprises a non-solid tumor or a solid tumor, such as acute leukemia, acute lymphoblastic leukemia, acute myeloid leukemia (including monocytic leukemia, myeloblastic leukemia, myelomonocytic leukemia or promyelocytic leukemia), acute T-cell leukemia, B-cell acute lymphoblastic leukemia, angiosarcoma, astrocytoma, basal cell carcinoma, cholangiocarcinoma, bladder cancer, brain cancer, breast cancer, bronchial cancer, prostate cancer, cervical cancer, chondrosarcoma, chordoma, choriocarcinoma, chronic leukemia, chronic lymphocytic leukemia, chronic myeloid leukemia or chronic myelocytic leukemia, chronic myelogenous leukemia, colon cancer, colorectal cancer or non-small cell lung cancer.
